# NEW EUROPEAN PATENT SPECIFICATION

(11) **EP 2 873 733 B2**
(45) Date of publication and mention of the opposition decision: **18.01.2023**
(45) Mention of the grant of the patent: 26.06.2019
(21) Application number: 13766387.8
(22) Date of filing: 09.07.2013
(51) Int. Cl.: C12N 15/74, A61K 39/02, C12R 1/35

(54) **VECTORS FOR TRANSFORMING MYCOPLASMA HYOPNEUMONIAE, TRANSFORMED M.HYOPNEUMONIAE STRAINS, AND USE THEREOF**
VEKTOREN ZUR TRANSFORMATION VON MYCOPLASMA HYOPNEUMONIAE, TRANSFORMIERTE STÄMME VON MYCOPLASMA HYOPNEUMONIAE, SOWIE DEREN VERWENDUNG
VECTEURS POUR LA TRANSFORMATION DE MYCOPLASMA HYOPNEUMONIAE, SOUCHES TRANSFORMANTES DE M.HYOPNEUMONIAE, ET LEUR UTILISATION

(30) Priority: 10.07.2012 EP 12382277
(43) Date of publication of application: 20.05.2015
(60) Divisional application: 19174710.4
(73) Proprietor: Hipra Scientific, S.L.U., E-17170 Amer (Girona) (ES)
(72) Inventor: GONZALEZ GONZALEZ, Luis, E-08915 Badalona (ES); PIÑOL RIBAS, Jaume, E-08197 Valldoreix (ES); MONTANE GIRALT, Jordi, E-17174 Sant Feliu De Pallerols (ES); CAMATS MALET, Maria, 08012 Barcelona (ES); QUEROL MURILLO, Enrique, E-08021 Barcelona (ES); SITJA ARNAU, Marta, E-17003 Girona (ES)
(74) Representative: Grünecker Patent- und Rechtsanwälte PartG mbB
(86) International application number: PCT/ES2013/070492
(87) International publication number: WO 2014/009586

(56) References cited:
- WO-A1-2009/061798
- Beatriz Machado Terra Lopes: "CONSTRUÇÃO DE VETOR ORIC DE MYCOPLASMA HYOPNEUMONIAE - UMA FERRAMENTA PARA ESTUDOS GENÉTICOS DO AGENTE DA PNEUMONIA ENZOÓTICA SUÍNA", LUME - Digital Repository of the Universidade Federal do Rio Grande do Sul , 2007, XP002689217, Porto Alegre Retrieved from the Internet: URL:http://www.lume.ufrgs.br/bitstream/han dle/10183/11473/000613564.pdf?sequence=1&l ocale=en [retrieved on 2012-12-14] cited in the application & TERRA LOPES BEATRIZ MACHADO: "Construction of a Mycoplasma hyopneumoniae oriC vector - a tool for genetic studies on the suine enzootic pneumonia agent", ACTA SCIENTIAE VETERINARIAE, vol. 37, no. 1, 2009, page 112, ISSN: 1678-0345(print)
- WEBER SHANA DE SOUTO ET AL: "Unveiling Mycoplasma hyopneumoniae Promoters: Sequence Definition and Genomic Distribution", DNA RESEARCH, vol. 19, no. 2, April 2012 (2012-04), pages 103-115, XP002689218,
- C. LARTIGUE ET AL: "Host specificity of mollicutes oriC plasmids: functional analysis of replication origin", NUCLEIC ACIDS RESEARCH, vol. 31, no. 22, 15 November 2003 (2003-11-15), pages 6610-6618, XP055047650, ISSN: 0305-1048, DOI: 10.1093/nar/gkg848
- S.-W. LEE ET AL: "Development of a replicable oriC plasmid for Mycoplasma gallisepticum and Mycoplasma imitans, and gene disruption through homologous recombination in M. gallisepticum", MICROBIOLOGY, vol. 154, no. 9, 1 September 2008 (2008-09-01), pages 2571-2580, XP055047652, ISSN: 1350-0872, DOI: 10.1099/mic.0.2008/019208-0
- VASCONCELOS ANA TEREZA R ET AL: "Swine and poultry pathogens: the complete genome sequences of two strains of Mycoplasma hyopneumoniae and a strain of Mycoplasma synoviae", JOURNAL OF BACTERIOLOGY, AMERICAN SOCIETY FOR MICROBIOLOGY, WASHINGTON, DC; US, vol. 187, no. 16, 1 August 2005 (2005-08-01), pages 5568-5577, XP002513991, ISSN: 0021-9193, DOI: 10.1128/JB.187.16.5568-5577.2005
- REDDY SHANKER P ET AL: "Isolation and characterization of transposon Tn4001-generated, cytadherence-deficient transformants of Mycoplasma pneumoniae and Mycoplasma genitalium", FEMS IMMUNOLOGY AND MEDICAL MICROBIOLOGY, vol. 15, no. 4, 1996, pages 199-211, XP002721523, ISSN: 0928-8244
- HEDREYDA C T ET AL: "Transformation of Mycoplasma pneumoniae with Tn4001 by Electroporation", PLASMID, NEW YORK,NY, US, vol. 30, no. 2, 1 September 1993 (1993-09-01), pages 170-175, XP024799159, ISSN: 0147-619X, DOI: 10.1006/PLAS.1993.1047 [retrieved on 1993-09-01]
- HALBEDEL ET AL: "Tools for the genetic analysis of Mycoplasma", INTERNATIONAL JOURNAL OF MEDICAL MICROBIOLOGY, URBAN UND FISCHER, DE, vol. 297, no. 1, 26 January 2007 (2007-01-26), pages 37-44, XP005863125, ISSN: 1438-4221, DOI: 10.1016/J.IJMM.2006.11.001
- ZIMMERMAN C U ET AL: "Synthesis of a small, cysteine-rich, 29 amino acids long peptide in Mycoplasma pneumoniae", FEMS MICROBIOLOGY LETTERS, WILEY-BLACKWELL PUBLISHING LTD, GB, vol. 253, no. 2, 15 December 2005 (2005-12-15), pages 315-321, XP027871759, ISSN: 0378-1097 [retrieved on 2005-12-15]
- B. MACHADO TERRA LOPES, THESIS FOR THE FEDERAL UNIVERSITY OF RIO GRANDE DO SUL, 2007, Porto Alegre, Brazil

### SEQUENCE LISTING

<110> HIPRA SCIENTIFIC, S.L.U.
<120> Mutant strains of Mycoplasma hyopneumoniae
<130> 102310PEP757L
<140> EP12382277.7
   <141> 2012-07-10
<160> 45
<170> PatentIn version 3.5
<210> 1
   <211> 265
   <212> DNA
   <213> Mycoplasma hyopneumoniae
<400> 1
   gaagactgat tagaaattta gaactattca aatctttcaa aaaagtggcc taaaaacaat      60
   gataaaaaaa tttccaaact tttttgttgc aaaaaaaaaa aaaaaaagta taattttaat     120
   tgtacaagtt aaataaattt ttcacttatc tttttttatt ttgcaaaact tttaaaaaaa     180
   ttagatatct aaattatatt atatgattga gaaaatgaaa aatttatttc ctagaaccaa     240
   gataattgag gtttaaaata tgagt                                           265
<210> 2
   <211> 263
   <212> DNA
   <213> Mycoplasma hyopneumoniae
<400> 2
   ttgaatttgt attttccata atctaaaatt ttacattttt ttataacaat ttttaaaaat      60
   taccctttaa tttatagtat ttttttattt tttagtctaa attataaaat tatcttgaat     120
   tttatttgaa tttttataat ttagtactaa aaaatacaaa tattttttcc tattctaaga     180
   aaaattcatt ttttaaaaaa aattgatttt tatagtataa tttatttgta taattgaatt     240
   aacttgattt gaaagggaac aaa                                             263
<210> 3
   <211> 699
   <212> DNA
   <213> Artificial Sequence
<220>
   <223> synthetic gene
<400> 3
   atgacatatc caagaagaag atatagaaga agaagacata gaccaagatc acatttagga      60
   caaattttaa gaagaagacc atgattagtt catccaagac atagatatag atgaagaaga     120
   aaaaatggaa tttttaatac aagattatca agaacatttg gatatacagt taaaagaaca     180
   acagttacaa caccatcatg agcagttgat atgatgagat ttaaaattga tgattttgtt     240
   ccaccaggag gaggaacaaa taaaatttca attccatttg aatattatag aattagaaaa     300
   gttaaagttg aattttgacc atgttcacca attacacaag gagatagagg agttggatca     360
   acagcagtta ttttagatga taattttgtt acaaaagcaa cagcattaac atatgatcca     420
   tatgttaatt attcatcaag acatacaatt ccacaaccat tttcatatca ttcaagatat     480
   tttacaccaa aaccagtttt agattcaaca attgattatt ttcaaccaaa taataaaaga     540
   aatcaattat gattaagatt acaaacatca ggaaatgttg atcatgttgg attaggaaca     600
   gcatttgaaa attcaaaata tgatcaagat tataatatta gagttacaat gtatgttcaa     660
   tttagagaat ttaatttaaa agatccacca ttagaacca                            699
<210> 4
   <211> 702
   <212> DNA
   <213> Artificial Sequence
<220>
   <223> synthetic gene
<400> 4
   atgacatatc caagaagaag atatagaaga agaagacata gaccaagatc acatttagga      60
   caaattttaa gaagaagacc atgattagtt catccaagac atagatatag atgaagaaga     120
   aaaaatggaa tttttaatac aagattatca agaacatttg gatatacagt taaaagaaca     180
   acagttacaa caccatcatg agcagttgat atgatgagat ttaaaattga tgattttgtt     240
   ccaccaggag gaggaacaaa taaaatttca attccatttg aatattatag aattagaaaa     300
   gttaaagttg aattttgacc atgttcacca attacacaag gagatagagg agttggatca     360
   acagcagtta ttttagatga taattttgtt acaaaagcaa cagcattaac atatgatcca     420
   tatgttaatt attcatcaag acatacaatt ccacaaccat tttcatatca ttcaagatat     480
   tttacaccaa aaccagtttt agattcaaca attgattatt ttcaaccaaa taataaaaga     540
   aatcaattat gattaagatt acaaacatca ggaaatgttg atcatgttgg attaggaaca     600
   gcatttgaaa attcaaaata tgatcaagat tataatatta gagttacaat gtatgttcaa     660
   tttagagaat ttaatttaaa agatccacca ttagaaccat aa                        702
<210> 5
   <211> 992
   <212> DNA
   <213> Artificial Sequence
<220>
   <223> synthetic gene
<400> 5
   actagtacta gagaagactg attagaaatt tagaactatt caaatctttc aaaaaagtgg      60
   cctaaaaaca atgataaaaa aatttccaaa cttttttgtt gcaaaaaaaa aaaaaaaagt    120
   ataattttaa ttgtacaagt taaataaatt tttcacttat ctttttttat tttgcaaaac     180
   ttttaaaaaa attagatatc taaattatat tatatgattg agaaaatgaa aaatttattt     240
   cctagaacca agataattga ggtttaaaat atgagtggat ccatgacata tccaagaaga     300
   agatatagaa gaagaagaca tagaccaaga tcacatttag gacaaatttt aagaagaaga     360
   ccatgattag ttcatccaag acatagatat agatgaagaa gaaaaaatgg aatttttaat     420
   acaagattat caagaacatt tggatataca gttaaaagaa caacagttac aacaccatca     480
   tgagcagttg atatgatgag atttaaaatt gatgattttg ttccaccagg aggaggaaca     540
   aataaaattt caattccatt tgaatattat agaattagaa aagttaaagt tgaattttga     600
   ccatgttcac caattacaca aggagataga ggagttggat caacagcagt tattttagat     660
   gataattttg ttacaaaagc aacagcatta acatatgatc catatgttaa ttattcatca     720
   agacatacaa ttccacaacc attttcatat cattcaagat attttacacc aaaaccagtt     780
   ttagattcaa caattgatta ttttcaacca aataataaaa gaaatcaatt atgattaaga     840
   ttacaaacat caggaaatgt tgatcatgtt ggattaggaa cagcatttga aaattcaaaa     900
   tatgatcaag attataatat tagagttaca atgtatgttc aatttagaga atttaattta     960
   aaagatccac cattagaacc ataagcggcc gc                                   992
<210> 6
   <211> 233
   <212> PRT
   <213> Porcine circovirus type 2 (PCV2)
<400> 6
   Met Thr Tyr Pro Arg Arg Arg Tyr Arg Arg Arg Arg His Arg Pro Arg
   1               5                   10                  15
   Ser His Leu Gly Gln Ile Leu Arg Arg Arg Pro Trp Leu Val His Pro
               20                  25                  30
   Arg His Arg Tyr Arg Trp Arg Arg Lys Asn Gly Ile Phe Asn Thr Arg
           35                  40                  45
   Leu Ser Arg Thr Phe Gly Tyr Thr Val Lys Arg Thr Thr Val Thr Thr
       50                  55                  60
   Pro Ser Trp Ala Val Asp Met Met Arg Phe Lys Ile Asp Asp Phe Val
   65                  70                  75                  80
   Pro Pro Gly Gly Gly Thr Asn Lys Ile Ser Ile Pro Phe Glu Tyr Tyr
                   85                  90                  95
   Arg Ile Arg Lys Val Lys Val Glu Phe Trp Pro Cys Ser Pro Ile Thr
               100                 105                 110
   Gln Gly Asp Arg Gly Val Gly Ser Thr Ala Val Ile Leu Asp Asp Asn
           115                 120                 125
   Phe Val Thr Lys Ala Thr Ala Leu Thr Tyr Asp Pro Tyr Val Asn Tyr
       130                 135                 140
   Ser Ser Arg His Thr Ile Pro Gln Pro Phe Ser Tyr His Ser Arg Tyr
   145                 150                 155                 160
   Phe Thr Pro Lys Pro Val Leu Asp Ser Thr Ile Asp Tyr Phe Gln Pro
                   165                 170                 175
   Asn Asn Lys Arg Asn Gln Leu Trp Leu Arg Leu Gln Thr Ser Gly Asn
               180                 185                 190
   Val Asp His Val Gly Leu Gly Thr Ala Phe Glu Asn Ser Lys Tyr Asp
           195                 200                 205
   Gln Asp Tyr Asn Ile Arg Val Thr Met Tyr Val Gln Phe Arg Glu Phe
       210                 215                 220
   Asn Leu Lys Asp Pro Pro Leu Glu Pro
   225                 230
<210> 7
   <211> 26
   <212> DNA
   <213> Staphylococcus aureus
<400> 7
   gataaagtcc gtataattgt gtaaaa 26
<210> 8
   <211> 26
   <212> DNA
   <213> Staphylococcus aureus
<400> 8
   ttttacacaa ttatacggac tttatc 26
<210> 9
   <211> 33
   <212> DNA
   <213> Artificial Sequence
<220>
   <223> primer
<400> 9
   atgcgcggcc gcttatttat cagaaacagt tag 33
<210> 10
   <211> 38
   <212> DNA
   <213> Artificial Sequence
<220>
   <223> primer
<400> 10
   agtcgggccc agcttgcgca tcattggatg atggattc 38
<210> 11
   <211> 29
   <212> DNA
   <213> Artificial Sequence
<220>
   <223> primer
<400> 11
   actgggatcc atgaatatag ttgaaaatg 29
<210> 12
   <211> 27
   <212> DNA
   <213> Artificial Sequence
<220>
   <223> primer
<400> 12
   ggatgggccc agcttgcgca tcattgg 27
<210> 13
   <211> 40
   <212> DNA
   <213> Artificial Sequence
<220>
   <223> primer
<400> 13
   gatctctaga tcgaggaaga ctgattagaa atttagaact 40
<210> 14
   <211> 34
   <212> DNA
   <213> Artificial Sequence
<220>
   <223> primer
<400> 14
   gatcggatcc actcatattt taaacctcaa ttat 34
<210> 15
   <211> 27
   <212> DNA
   <213> Artificial Sequence
<220>
   <223> primer
<400> 15
   actgactagt agcttgcgca tcattgg 27
<210> 16
   <211> 43
   <212> DNA
   <213> Artificial Sequence
<220>
   <223> primer
<400> 16
   atgcagatct atgtccaatt tactgaccgt acaccaaaat ttg 43
<210> 17
   <211> 37
   <212> DNA
   <213> Artificial Sequence
<220>
   <223> primer
<400> 17
   atgcgggccc ttaatcgcca tcttccagca ggcgcac 37
<210> 18
   <211> 45
   <212> DNA
   <213> Artificial Sequence
<220>
   <223> primer
<400> 18
   tgttatcgat accgtcgatg cacctcgagc taagttattt tattg 45
<210> 19
   <211> 44
   <212> DNA
   <213> Artificial Sequence
<220>
   <223> primer
<400> 19
   agacatcgaa agcttgatta tgctgattgc attctttcaa tttg 44
<210> 20
   <211> 42
   <212> DNA
   <213> Artificial Sequence
<220>
   <223> primer
<400> 20
   agaggaatcc gatttattta tcagaaacag ttagtctttt cc 42
<210> 21
   <211> 58
   <212> DNA
   <213> Artificial Sequence
<220>
   <223> primer
<400> 21
   agactctaga actagtggat cccccgggcc cctcgaggaa gactgattag aaatttag 58
<210> 22
   <211> 46
   <212> DNA
   <213> Artificial Sequence
<220>
   <223> primer
<400> 22
   agacgaattc gaattcctgc agggatccac tcatatttta aacctc 46
<210> 23
   <211> 34
   <212> DNA
   <213> Artificial Sequence
<220>
   <223> primer
<400> 23
   catcgaattc acgaattagt gattctgcct tttc 34
<210> 24
   <211> 35
   <212> DNA
   <213> Artificial Sequence
<220>
   <223> primer
<400> 24
   catcgaattc attaaagttg attcggtgtt taatc 35
<210> 25
   <211> 35
   <212> DNA
   <213> Artificial Sequence
<220>
   <223> primer
<400> 25
   catgctgcag cccgggggat ccactagttc tagag 35
<210> 26
   <211> 27
   <212> DNA
   <213> Artificial Sequence
<220>
   <223> primer
<400> 26
   gtacccaatt cgccctatag tgagtcg 27
<210> 27
   <211> 30
   <212> DNA
   <213> Artificial Sequence
<220>
   <223> primer
<400> 27
   tcgaggaaga ctgattagaa atttagaact 30
<210> 28
   <211> 40
   <212> DNA
   <213> Artificial Sequence
<220>
   <223> primer
<400> 28
   gatcgggccc tcgaggaaga ctgattagaa atttagaact 40
<210> 29
   <211> 39
   <212> DNA
   <213> Artificial Sequence
<220>
   <223> primer
<400> 29
   gatcggatcc atgacccaag tacattttac actgaaaag 39
<210> 30
   <211> 39
   <212> DNA
   <213> Artificial Sequence
<220>
   <223> primer
<400> 30
   gatcctcgag ggggggccct tttacacaat tatacggac 39
<210> 31
   <211> 34
   <212> DNA
   <213> Artificial Sequence
<220>
   <223> primer
<400> 31
   gatcggatcc atgaaaatta ttaatattgg agtt 34
<210> 32
   <211> 50
   <212> DNA
   <213> Artificial Sequence
<220>
   <223> primer
<400> 32
   gatcgctgca ggaattcgat atcaagctta tcgataccgt cgatgcacct 50
<210> 33
   <211> 34
   <212> DNA
   <213> Artificial Sequence
<220>
   <223> primer
<400> 33
   ataacttcgt atagcataca ttatacgaac ggta 34
<210> 34
   <211> 69
   <212> DNA
   <213> Artificial Sequence
<220>
   <223> primer
<400> 34
   gattaagggc ccataacttc gtataggata ctttatacga agttatgtcg accccctcga 60
   ggaagactg 69
<210> 35
   <211> 74
   <212> DNA
   <213> Artificial Sequence
<220>
   <223> primer
<400> 35
   gggactagtt acggttcgta taatgtatgc tatacgaagt tatctgcagg atatcaagct 60
   tatcgatacc gtcg 74
<210> 36
   <211> 34
   <212> DNA
   <213> Artificial Sequence
<220>
   <223> primer
<400> 36
   gtgtaagctt aaaaaccagg atgcacaaaa taac 34
<210> 37
   <211> 34
   <212> DNA
   <213> Artificial Sequence
<220>
   <223> primer
<400> 37
   tgttgcggcc gctttaggca tcaggattat caac 34
<210> 38
   <211> 33
   <212> DNA
   <213> Artificial Sequence
<220>
   <223> primer
<400> 38
   agacactagt ttgaatttgt attttccata atc 33
<210> 39
   <211> 33
   <212> DNA
   <213> Artificial Sequence
<220>
   <223> primer
<400> 39
   agagggatcc tgtaattgtt gaagttgctg cct 33
<210> 40
   <211> 33
   <212> DNA
   <213> Artificial Sequence
<220>
   <223> primer
<400> 40
   tgttgcggcc gcttatggtt ctaatggtgg atc 33
<210> 41
   <211> 31
   <212> DNA
   <213> Artificial Sequence
<220>
   <223> primer
<400> 41
   gtgtggatcc atgacatatc caagaagaag a 31
<210> 42
   <211> 44
   <212> DNA
   <213> Artificial Sequence
<220>
   <223> primer
<400> 42
   atcttcttct tggatatgtc atggcatcag gattatcaac atta 44
<210> 43
   <211> 44
   <212> DNA
   <213> Artificial Sequence
<220>
   <223> primer
<400> 43
   taatgttgat aatcctgatg ccatgacata tccaagaaga agat 44
<210> 44
   <211> 31
   <212> DNA
   <213> Artificial Sequence
<220>
   <223> primer
<400> 44
   atgagtggat ccatgaaaat tattaatatt g 31
<210> 45
   <211> 36
   <212> DNA
   <213> Enterococcus faecalis
<400> 45
   ctaagttatt ttattgaaca tatatcgtac tttatc 36

## Description

### Technical Field

The present invention is comprised in the field of the development of methods and tools for the genetic manipulation of *Mycoplasma hyopneumoniae* for the purpose of preparing transformed mutant strains of said bacterial species which can be used as vaccines against porcine diseases.

### Background Art

In the veterinary field, diseases caused by microorganisms such as bacteria, viruses or parasites cause serious economic losses due to death of the animals, or due to a low performance in the growth and fattening processes.

One of the ways to protect animals against future infections consists of the administration of vaccines to generate an immunological response.

Vaccines based on inactivated microorganisms or live attenuated microorganisms have been the main immunological basis for the control and eradication of most infectious diseases up until now.

However, despite the good results obtained with these types of vaccines, they present problems such as the possible reversion to the virulence of attenuated vaccines, the inability to differentiate vaccinated animals from infected animals or the difficulty of achieving a vaccine that is effective for all diseases.

The development of tools for the genetic manipulation of microorganisms has typically allowed preparing vaccines that are more effective than those mentioned above, such as for example, subunit vaccines, genetically modified inactivated or live vaccines, marker vaccines (DIVA) or DNA vaccines.

Nevertheless, not all the disease-causing microorganisms in the veterinary field can be easily genetically manipulated.

Mycoplasmas are among the microorganisms difficult to be manipulated at the genetic level.

Mycoplasmas belong to the *Mollicutes* class, a wide group of microorganisms closely related with Gram positive bacteria.

Mycoplasmas have small circular genomes with sizes generally smaller than 1,000 kb, with a number of genes ranging between 500 and 1000.

This group of bacteria also lacks many of the metabolic pathways that are usually found in all living organisms, probably as a result of their adaptation to a parasitic lifestyle. In consequence, mycoplasmas are fastidious microorganisms that are very difficult to culture both in liquid and in solid medium.

Mycoplasmas include *Mycoplasma hyopneumoniae* (hereinafter, *"Mhyo*"), which is a bacterium responsible for huge economic losses in the porcine industry. Although most Mhyo infections are not severe, animals infected by *Mhyo* are predisposed to secondary infections of the respiratory system that can reduce the daily weight gain rate or even cause death.

*Mhyo* is the causative agent of porcine enzootic pneumonia (hereinafter "PEP"), a very widespread worldwide chronic respiratory disease. The disease is characterized by high morbidity and low mortality. The prevalence of PEP is particularly high in fattening pigs and the severity of the clinical signs depends on strain of *Mhyo* causing the infection, on environmental conditions, on the health condition of the animals and on the onset of secondary infections due to other pathogenic agents. In some cases, PEP presents in a sub-clinical manner without evident clinical signs. In cases where there are no complications, the animals show a chronic, non-productive cough associated with a reduced daily weight gain rate, with the subsequent reduction of the feed conversion efficiency. When secondary infections occur, the clinical signs become more evident, presenting acute respiratory symptoms and pyrexia, which can even cause the death of the animal. Nevertheless, even in the absence of complications due to secondary infections, PEP causes serious economic losses due to the low feed conversion rates and the costs derived from medication. Once the disease is established on a farm, it is transmitted horizontally to other animals by means of the cough-generated aerosols and vertically from the reproductive females to the piglets.

In addition to problematic culturing, the genetic manipulation of mycoplasmas is hindered because of the few tools available.

The difficulties in the genetic manipulation of mycoplasmas, particularly of *Mhyo,* have been described in the state of the art.

The genome sequence of *Mhyo* strain 232 is described in the article by Minion et al., The Genome Sequence of Mycoplasma hyopneumoniae Strain 232, the Agent of Swine Mycoplasmosis, J. Bacteriol., 2004, 186(21), 7123-7133. The author states that *Mhyo* is a fastidious microorganism and that there is a lack of tools and protocols for transforming it.

In the context of the study of modifications occurring in *Mhyo* when subjected to a temperature change, in the article by Madsen et al., Transcriptional Profiling of Mycoplasma hyopneumoniae during Heat Shock Using Microarrays, Infect. Immun., 2006, 74(1), 160-166, the authors state that despite the huge importance of *Mhyo* in porcine production, there have been few studies on the potential molecular mechanisms of its pathogenesis in response to environmental changes, and that this is probably due to its growth difficulties and due to the fact that it is a microorganism refractory to genetic manipulation.

In the article by Pich et al., Comparative analysis of antibiotic resistance marker genes in Mycoplasma genitalium: application to studies of the minimal gene complement, Microbiology, 2006, 152, 519-527, the genetic modification of *Mycoplasma genitalium* with different plasmids by electroporation is described, but no reference to *Mhyo* can be found.

The dissertation by B. Machado, Construçao de vetor oriC de Mycoplasma hyopneumoniae - uma ferramenta para estudos genéticos do agente da Pneumonia Enzoótica Suína, Porto Alegre, 2007, describes the transformation of the *Mhyo* strain 7448 with the replicative plasmid pOSTM by electroporation. To construct this plasmid, the *oriC* region of *Mhyo* and the expression cassette containing the tetracycline resistance gene (*tetM*), under the control of the *Spiroplasma citri* promoter gene, were introduced in the pUC18 vector. While the experimental part shows by means of PCR, the incorporation of the plasmid and the antibiotic resistance conferred by said vector, no conclusive results showing that the plasmid pOSTM was actually stably incorporated into *Mhyo* are provided in this work, since the isolation of the plasmids from the tetracycline-resistant transforming strains is not described. Furthermore, it is also described that the transforming strains had difficulties in growing after two passes in a selective medium. Consequently, these results show that using the transformation disclosed in this document it could not be possible to stably introduce an exogenous DNA sequence in a strain of *Mhyo,* since it cannot be recovered after multiple generations of the transformed bacteria.

Despite this disclosure in 2007, it has not been shown that the problem of the genetic modification of *Mhyo* was solved in subsequent studies.

In the report by M. Calcutt on the project entitled Development of genetic manipulation protocols for Mycoplasma hyopneumoniae clade of animal pathogens, University of Missouri, corresponding to the period 01.10.2009 to 30.09.2010 (downloaded from the web page http://www.reeis.usda.gov/web/ crisprojectpages/220630.html, retrieved on 16.02.2012), it is described that the objectives of the project is to develop methodology for the genetic manipulation of *Mhyo.* It is also described that it was intended to electrotransform *Mhyo* with plasmids including the tetracycline resistance gene. However, the results from the DNA analysis performed on the transforming bacteria were not positive and led the authors to consider that the transformation had not occurred. The results on the expression of this resistance gene were also not described.

The article by Browning et al., Developing attenuated vaccines to control mycoplasmoses, Microbiology Australia, 2011, 121-122, describes the development of vaccines containing temperature-sensitive strains of *Mycoplasma* that do not grow at the host's body temperature. Said strains were obtained by chemical mutation from wild type strains. The authors state that the directed genetic manipulation in *Mhyo* and *M. synoviae* continues to be a challenge and that it had not been achieved in said species. International patent application WO-A-2010/132932, from the same work group, also relates to temperature-sensitive strains of *Mhyo.*

In the article by Maboni et al., Mycoplasma hyopneumoniae Transcription Unit Organization: Genome Survey and Prediction, DNA Research, 2011, 18, 413-422, a comparative review of the genome of three strains of *Mhyo* (7448, J and 232) was conducted for the purpose of identifying open reading frames. The authors state that in spite of the development of the artificial transformation and other genetic tools for some species of *Mycoplasma,* these methodologies are yet to be developed for *Mhyo.*

Therefore, methods for the transformation of *Mhyo* have not been described in the state of the art, though there have been several unsuccessful attempts to perform such transformation.

Therefore, there is still a need to provide a method for preparing mutant strains of *Mhyo* by means of genetic engineering tools, thus providing mutant strains suitable for preparing vaccine compositions against porcine enzootic pneumonia and, eventually in combination with other antigen components, for the prevention and/or treatment of other porcine diseases.

### Disclosure of the Invention

The object of the present invention is a method for preparing mutant strains of *Mhyo.*

Another object of the invention is a replicative plasmid vector used in said method.

Another object of the invention is the use of said vector to prepare mutant strains of *Mhyo.*

Another object of the invention is a mutant strain of *Mhyo* obtainable by said method.

Another object of the invention is a mutant strain of *Mhyo* comprising an exogenous DNA sequence.

Another object of the invention is a mutant strain of *Mhyo* comprising the vector of the invention.

Another object of the invention is a strain of *Mhyo* transformed with the vector of the invention.

Another object of the invention is the use of said mutant strain as an expression host.

Another object of the invention is a vaccine comprising said mutant strain.

Another object of the invention is the use of the mutant strain of *Mhyo* of the invention for preparing a vaccine.

Another object of the invention is a vaccination kit comprising said mutant strain.

The authors of the present invention have developed a method for preparing mutant strains of *Mhyo* whereby exogenous DNA sequences can surprisingly be stably introduced into the cytoplasm of a strain of *Mhyo* and the content of those sequences is expressed.

By the expression "an exogenous DNA sequence stably introduced into the cytoplasm of a strain of Mhyo" it is understood that such sequence is not lost along the successive generations of the bacterium that has been transformed with said exogenous sequence. This stability means that the exogenous DNA sequence can replicate inside the transformed bacterium and in its descendants, and that it can be recovered after multiple generations of the transformed bacterium.

With the developed method, it is open a door to modifying strains of *Mhyo* for the first time by means of genetic engineering tools.

Mutant strains of *Mhyo* including exogenous DNA sequences can be prepared with said method. Said mutant strains can have different characteristics such as, for example, expressing a specific protein, or having a lower degree of virulence with respect to the wild type parental strain.

Said mutant strains can be used as vaccines, for example, live attenuated vaccines, inactivated vaccines, marker vaccines, which allow differentiating vaccinated animals from infected animals, or polyvalent vaccines against porcine enzootic pneumonia and another additional disease that can affect pigs, such as infections caused by porcine circovirus type 2 (PCV2), for example.

The process of the invention allowed for the first time to modify genetically *Mhyo* in stable form, and therewith it has been achieved, inter alia, the use of these transformed strains as expression vector of exogenous DNA sequences like, for example, nucleotide sequences coding for proteins of therapeutic or preventive interest such as the sequence of the PCV2 capsid, among others. By means of the technology disclosed in this invention, new vaccine candidates have been designed and prepared, which are also polyvalent, because they can be used to prevent different diseases by administering a single strain modified by means of the process of the invention.

In the present description and in the claims, the singular forms "a", "an" and "the" include plural references unless otherwise clearly indicated by the context.

Likewise, in the context of the invention the term "sequence" refers to a DNA sequence, except when it is explicitly indicated that it refers to an amino acid sequence.

### Description of the Drawings

Figure 1

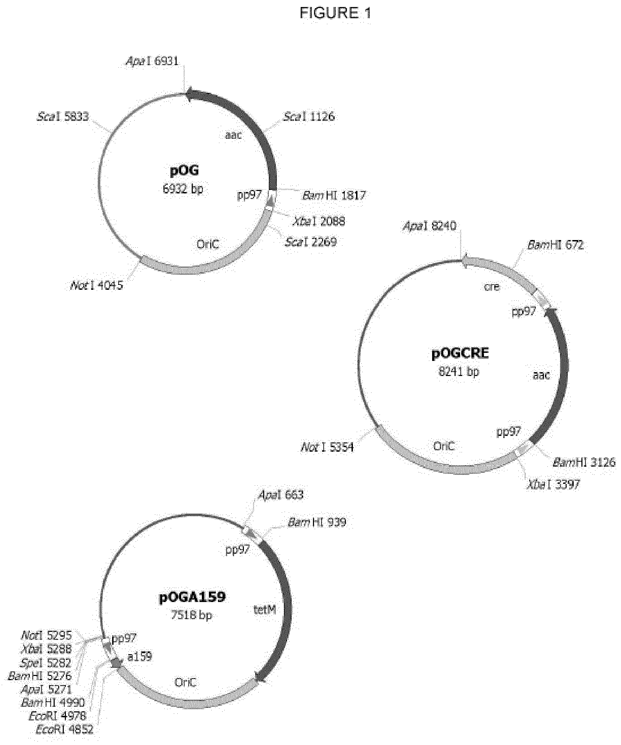

   Figure 1 shows a diagram of the replicative plasmids used to genetically modify *Mhyo* and achieve the production of recombinant proteins or antisense transcripts. The promoters, the genes and the restriction enzyme sites used for cloning operations are indicated by arrows.
   The plasmid pOG contains the necessary elements so that this vector can replicate in *Mhyo* at the same time that allows the selection of the carrier cells of said plasmid. This plasmid has the *oriC* region of *Mhyo* (bases 2096 to 4043) and the gentamicin resistance marker gene (*aac*, bases 1 to 1816) under the control of the promoter of the gene of the *Mhyo* P97 protein (*pp97,* bases 1823 to 2087).
   The plasmid pOGCRE derives directly from plasmid pOG and furthermore contains the cre gene of the phage P1 (bases 1 to 1032) under the control of the promoter *pp97* (bases 1039-1303).
   Unlike the previous plasmids, plasmid pOGA159 contains the tetracycline resistance gene (*tetM,* bases 945 to 2883) under the control of the promoter *pp97* (bases 674-938). The *oriC* region of *Mhyo* is located between bases 2901-4848. Finally, it contains a segment of the *Mhyo* genome corresponding to the 5' end of the *tlyA* gene (*a159*, bases 4855 to 4980) reversely oriented and under the control of the promoter *pp97* (bases 4996-5260).
Figure 2

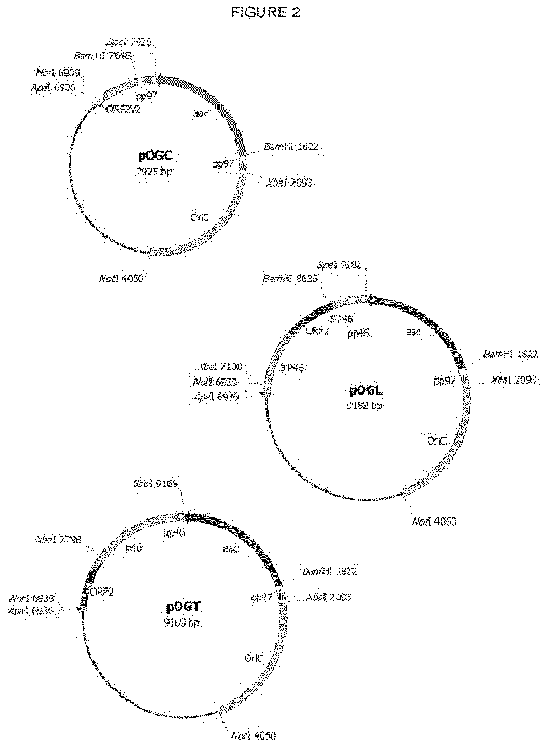

   Figure 2 shows a diagram of the replicative plasmids used to genetically modify *Mhyo* and to achieve the production of different recombinant versions of the protein of the PCV2 virus capsid. The promoters, the genes and the restriction enzyme sites used for cloning operations are indicated by arrows.
   The plasmid pOGC contains exactly the same elements as the plasmid pOG (Figure 1) and furthermore includes an additional copy of the promoter *pp97* (bases 7654-7918) controlling the expression of the synthetic gene coding for the circovirus capsid included in the ORF2V2 (bases 6946-7647) of the PCV2 virus capsid. This plasmid allows the expression of the PCV2 capsid protein in the cytoplasm of the carrier cell.
   The plasmid pOGL contains exactly the same elements as the plasmid pOG (Figure 1) and furthermore includes the promoter sequence of the *Mhyo* P46 protein gene (*pp46:* bases 8918-9181) controlling the expression of a fusion of the gene of the protein of the PCV2 virus capsid (ORF2) inside of the gene coding for the P46 *Mhyo* membrane protein (bases 8642-8917 and 6946-7930). This plasmid produces a hybrid protein which is characterized in that amino acids 1 to 92 are from the N-terminal end of the P46 protein, amino acids 95 to 327 correspond to the protein of the PCV2 virus capsid and amino acids 330-656 are from the C-terminal end of the *Mhyo* P46 protein.
   The plasmid pOGT contains exactly the same elements as the plasmid pOG (Figure 1) and furthermore includes the promoter sequence *pp46* (bases 8905-9169) controlling the expression of a fusion of the gene of the protein of the PCV2 virus capsid (ORF2) at the 3' end of the gene coding for the P46 *Mhyo* membrane protein (bases 7648-8904). This plasmid produces a hybrid protein which is characterized in that amino acids 1 to 419 are from the P46 protein and amino acids 420 to 652 correspond to the protein of the PCV2 virus capsid, ORF2 version (bases 6946-7647).

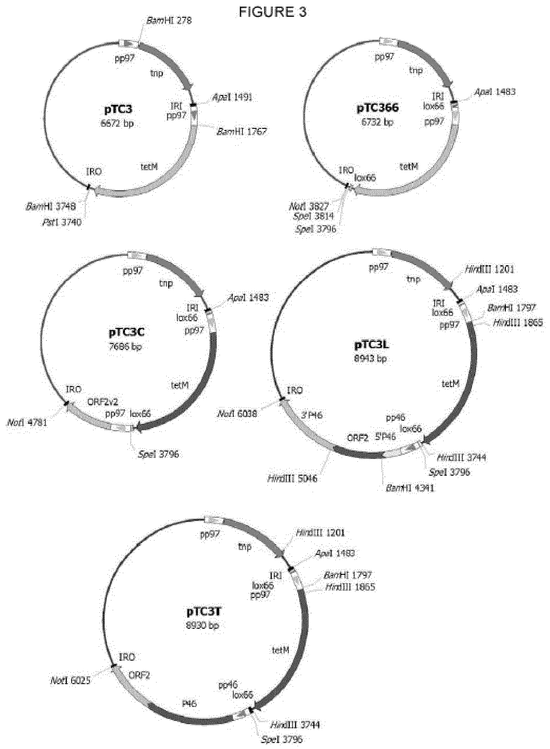

Figure 3 (not part of the invention)
   Figure 3 shows a diagram of the plasmids carrying minitransposons used to obtain insertions in the *Mhyo* chromosome. The promoters, genes and targets of restriction enzymes used for the construction of the plasmids and other important DNA sequences are indicated by arrows.
   The plasmid pTC3 contains the elements necessary for obtaining strains of *Mhyo* bearing transposon insertions. This plasmid has been constructed on the backbone of the plasmid pMTn4001. The first element of this plasmid is the promoter *pp97* (bases 7-271) controlling the expression of the transposase of plasmid pMTn4001 (bases 284-1261). Bases 1461-1486 and 3774-3799 correspond to the inverted repeats and are indicated in the diagram as IRI and IRO, respectively. The last element of this plasmid corresponds to the tetracycline resistance marker gene (bases 1761-3692), which is also under the control of the promoter of the *Mhyo* P97 protein (1502-1766).
   Plasmid pTC366 derives directly from plasmid pTC3 and contains the *lox66* sequences (substrate of the Cre recombinase, bases 1485-1518 and 3762-3795) flanking the tetracycline resistance marker gene *TetM* (bases 1791-3722).
   Plasmid pTC3C derives directly from plasmid pTC366 and contains the gene coding for the protein of the PCV2 virus capsid *ORF2V2* (bases 4078- 4779) of the PCV2 virus, under the control of the promoter *pp97* (bases 3808-4071). This plasmid promotes the synthesis of the recombinant protein corresponding to the PCV2 capsid protein in the cytoplasm of the strain of *Mhyo* transformed by said plasmid.
   Plasmid pTC3L derives directly from plasmid pTC366 and contains a fusion of the gene of the protein of the PCV2 virus capsid (*ORF2*) inside of the gene coding for the P46 *Mhyo* membrane protein (bases 4347-5045 and 4066-4340 and 5052-6036, respectively), under the control of the promoter *pp46* (bases 3801-4065). This plasmid promotes the synthesis of a hybrid protein, which is characterized in that amino acids 1 to 92 are from the N-terminal end of the P46 protein, amino acids 95 to 327 correspond to the protein of the PCV2 virus capsid and amino acids 330-656 are from the C-terminal end of the *Mhyo* P46 protein.
   Plasmid pTC3T derives directly from plasmid pTC366 and contains a fusion between the gene of the protein of the PCV2 virus capsid (*ORF2*) and the gene coding for the P46 *Mhyo* membrane protein (bases 5322-6031 and 4065-5321, respectively), under the control of the promoter of the gene coding for the *Mhyo* P46 protein (bases 3801-4065). This plasmid promotes the synthesis of a hybrid protein which is characterized in that amino acids 1 to 419 are from the P46 protein and amino acids 420 to 652 correspond to the protein of the PCV2 virus capsid.
Figure 4

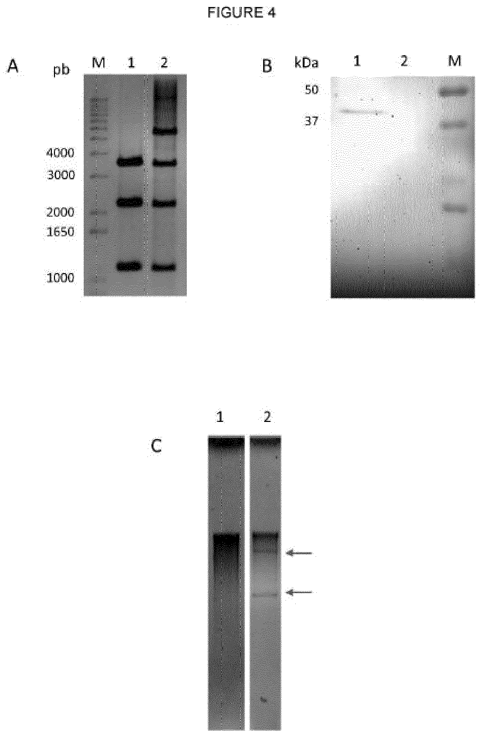

   Figure 4 shows the stability of the replicative plasmids and their use in the production of recombinant proteins.
   This figure is split into 3 panels. Panel A shows a 0.7% agarose gel electrophoresis run at a voltage of 6V/cm for 2 hours. The 1kb plus ladder molecular weight marker (Invitrogen) is in lane M. The plasmid pOG isolated from E. *coli* XI1-blue and digested with restriction enzyme *Sca*I is in lane 1. A total DNA extraction of the *Mhyo* strain 232POGc9 digested with restriction enzyme *Sca*I is in lane 2. The expected bands of the plasmid pOG digested with restriction enzyme *Sca*I are 3564 bp, 2225 bp and 1143 bp (Figure 4, Panel A). By means of the comparison with the molecular weight marker and the bands of the plasmid isolated from E. *coli* XI1-blue, it is confirmed that the plasmid pOG propagates stably in the strain 232POGc9. The lengths of the different relevant DNA marker fragments are indicated on the left side of the figure.
   Panel B shows a Western blot of a denaturing polyacrylamide gel electrophoresis with protein samples of different strains of *Mhyo* detected by means of a specific polyclonal antibody of the Cre recombinase of the bacteriophage P1. Lane 1 shows a protein extract of strain 232POGCREc1 where the expected band of 39 kDa is observed. Lane 2 shows an extract of the strain 232 where no bands are observed and the Precision Plus Protein Dual Xtra (Biorad) molecular weight marker is in lane M. The relevant molecular weights are shown in kDa on the right side of the figure. These results indicate that the plasmid pOGCRE is capable of producing the Cre protein once it is transformed into *Mhyo* strain 232.
   Panel C shows an agarose gel electrophoresis in which DNA samples of *Mhyo* parental strain 6314 (lane 1) and of strain 6314pOGAc4, which is a product of the transformation of strain 6314 with the plasmid pOGA159 (lane 2), are analyzed. The arrows indicate the position of the two conformations adopted by plasmid pOGA159 in the transformed mutant strain of *Mhyo.*

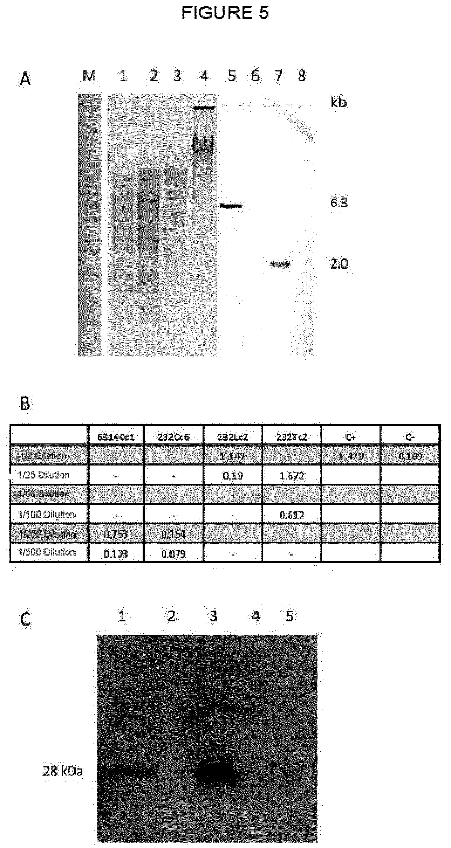

Figure 5 (not part of the invention)
   Figure 5 shows the recovery of *Mhyo* strains bearing transposon insertions and the usefulness thereof in the production of recombinant proteins.
   This figure is split into 3 panels. Panel A (lanes M and 1 to 4) shows a 0.7% agarose gel electrophoresis run at a voltage of 6V/cm for 2 hours whereas the right side of panel A (lanes 5 to 8) shows a Southern blot of this gel on a nylon membrane. The bands observed in the Southern blot correspond to the bands recognized by a probe designed against the *TetM* sequence under the control of the promoter of the *Mhyo* P97 protein. The 1kb Plus Ladder (Invitrogen) molecular weight marker is in lane M. Lanes 1 and 5 correspond to a total DNA extraction from strain 232TC3hlyC digested with restriction enzyme *Eco*RI. Lanes 2 and 6 correspond to a total DNA extraction from strain 232 digested with restriction enzyme *Eco*RI. Lanes 3 and 7 correspond to a total DNA extraction of strain 232TC3hlyC digested with restriction enzyme *Eco*RV. Lanes 4 and 8 correspond to a total DNA extraction of strain 232 digested with *Eco*RV. The band of 6.3 kb in lane 5 and the band of 2 kb in lane 7 demonstrate the presence of the transposon containing the *TetM* resistance under the promoter of the P97 protein in the *Mhyo* genome whereas the absence of these bands in lanes 6 and 8 corroborate these results.
   Panel B shows the results of an ELISA assay performed with the INGEZIM PCV DAS kit (Ingenasa, Spain) with different protein samples from *Mhyo.* The dilution used in each of the assays is indicated in the left column of the table, whereas the strain analyzed is shown in the upper row. The numerical results are the values from absorbance readings at 450 nm wavelength normalized by the amount of protein introduced in each well. All the strains of *Mhyo* transformed with the pTC3C, pTC3L and pTC3T vectors exhibit an absorbance that is clearly higher than that of the negative control, which indicates that the expression of the recombinant PCV2 capsid protein takes place in each one of these strains.
   The panel C shows a Western blot of a denaturing polyacrylamide gel electrophoresis with protein samples of different strains of *Mhyo* detected by means of a specific monoclonal antibody of the PCV2 capsid protein. Lane 1 contains a protein extract of strain 232Cc6 where the expected band of 28 kDa is observed. Lane 2 contains a protein extract of strain 232 where no bands are observed. Lane 3 contains a protein sample of the PCV2 capsid supplied as a positive control in the INGEZIM PCV DAS kit (Ingenasa, Spain) where the expected band of 28 kDa is observed. Lane 4 contains a protein extract of strain 6314 where no bands are observed, whereas the protein extract of strain 6314Cc1 is in lane 5 where the expected band of 28 kDa is observed, though this band has a weaker intensity than those of lanes 1 and 3. These results indicate that in both 6314 and 232 strains the insertion in the *Mhyo* genome of the transposon present in the plasmid pTC3C induces expression of the PCV2 capsid protein, coded by *ORF2v2.*

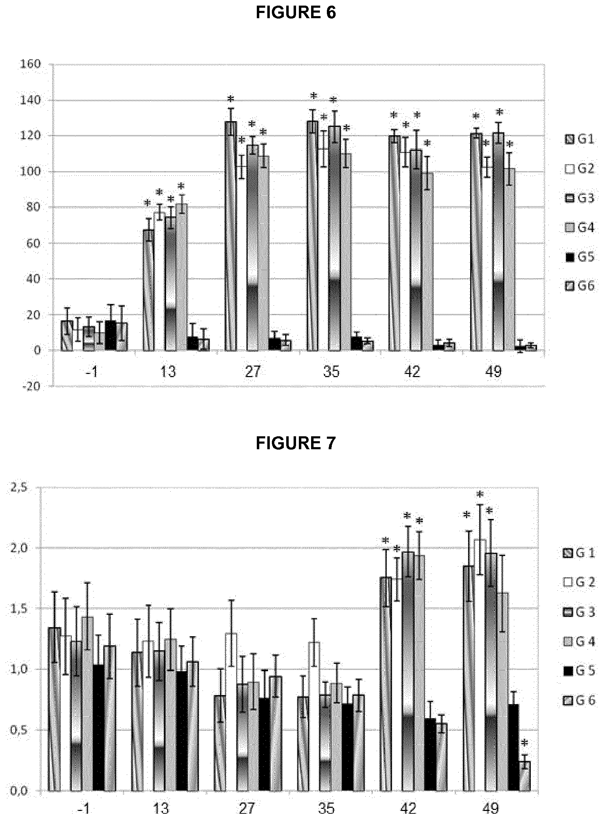

Figure 6 (not part of the invention)
   Figure 6 shows the serological response to *Mhyo* obtained with the experimental vaccines, groups 1 to 4 of the Example 7.1. Groups 5 and 6 correspond respectively to the non-vaccinated, but infected control group and to the non-vaccinated and non-infected control group.
   The day of the study when the analysis was performed is on the x-axis, and the S/P ratio (sample/positive) expressed as the % measured by ELISA is represented on the y-axis as an indicator of the IgG antibody response against *Mhyo*. The asterisk indicates that the difference in relation to the non-vaccinated, but infected control group is statistically significant (p<0.05, according to the Mann-Whitney U test).
Figure 7 (not part of the invention)
   Figure 7 shows the serological response to porcine circovirus (PCV2) obtained with the experimental vaccines, groups 1 to 4 of Example 7.1. Groups 5 and 6 correspond respectively to the non-vaccinated, but infected control group and to the non-vaccinated and non-infected control group.
   The day of the study when the analysis was performed is on the x-axis, and the S/P ratio (sample/positive) expressed as the % measured by ELISA is represented on the y-axis as an indicator of the IgG antibody response against PCV2. The asterisk indicates that the difference in relation to the non-vaccinated but infected control group is statistically significant (p<0.05, according to the one-way ANOVA test).

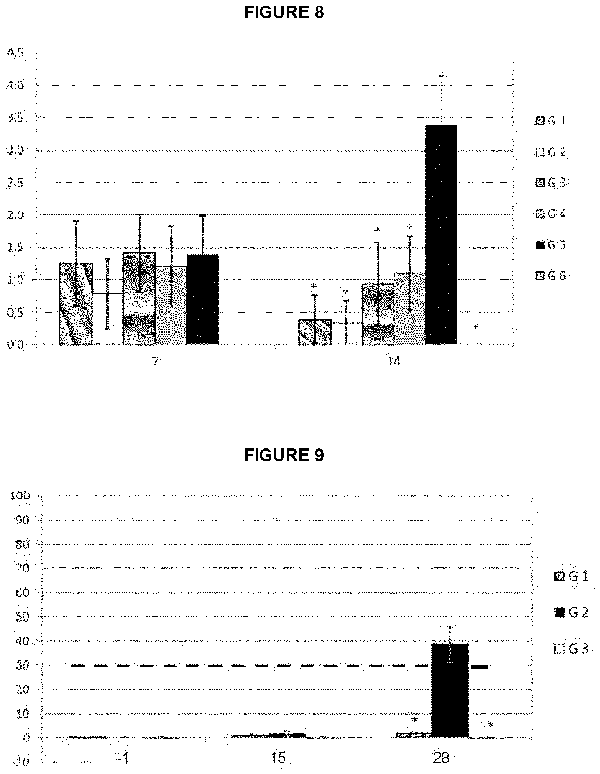

Figure 8 (not part of the invention)
   Figure 8 shows the serum viral load in treatment groups 1 to 4, and in the control groups 5 and 6 of Example 7.1. The day of the study when the analysis was performed is on the x-axis, and the log₁₀ of the serum viral load of porcine circovirus type 2 (PCV2) expressed in copies/ml is represented on the y-axis.
   The asterisk indicates that the difference in relation to the control group (non-vaccinated and infected) is statistically significant (p<0.05, according to the Mann-Whitney U test).
Figure 9
   Figure 9 shows the serological response to infection by *Mhyo* according to Example 8.2. Group 1 corresponds to the animals infected with the mutant strain of Example 4.8, group 2 corresponds to the animals infected with the wild type parental strain, and group 3 is the non-infected control group.
   The day of the study when the analysis was performed is on the x-axis, and the S/P ratio (sample/positive) expressed as the % measured by ELISA is represented on the y-axis, as an indicator of the IgG antibody response against *Mhyo.* The asterisk indicates that the difference in relation to the group infected with the wild type parental strain is statistically significant (p<0.05, according to the one-way ANOVA test).
Figure 10

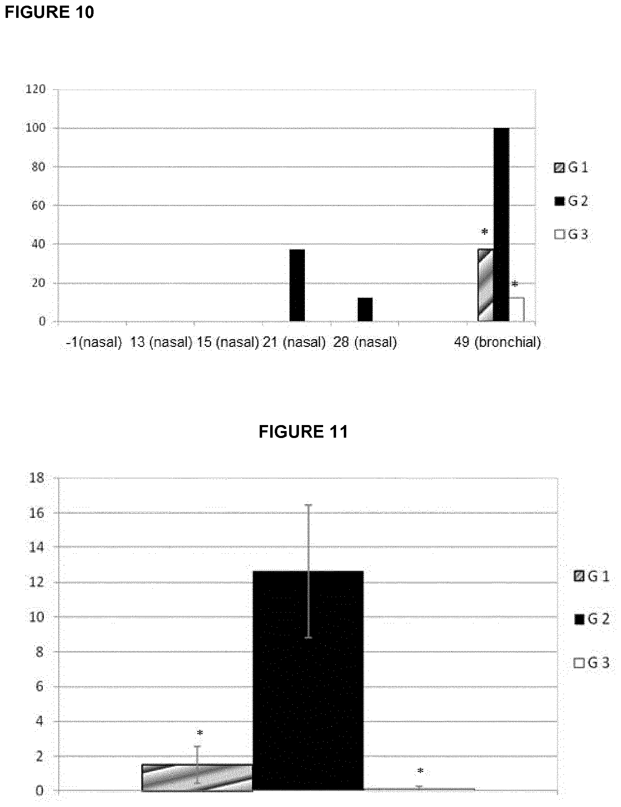

   Figure 10 shows the percentage of *Mhyo PCR*-positive animals of Example 8.2. Group 1 corresponds to the animals infected with the mutant strain of Example 4.8, group 2 corresponds to the animals infected with the wild type parental strain, and group 3 is the non-infected control group.
   The day of the study when the analysis was performed is on the x-axis as is the type of sample analyzed (nasal or bronchial), and the percentage of PCR-positive animals is represented on the y-axis.
   The asterisk indicates that the difference in relation to the group infected with the wild type parental strain is statistically significant (p<0.05, according to Fisher's exact test).
Figure 11
   Figure 11 shows the mean surface of lung affected by lesions which can be attributed to infection by *Mhyo* according to the assay of Example 8.2. Group 1 corresponds to the animals infected with the mutant strain of Example 4.8, group 2 corresponds to the animals infected with the wild type parental strain, and group 3 is the non-infected control group.
   The mean value of the affected lung surface is represented on the y-axis.
   The asterisk indicates that the difference in relation to the group infected with the wild type parental strain is statistically significant (p<0.05, according to the Mann-Whitney U test).
Figure 12 (not part of the invention)

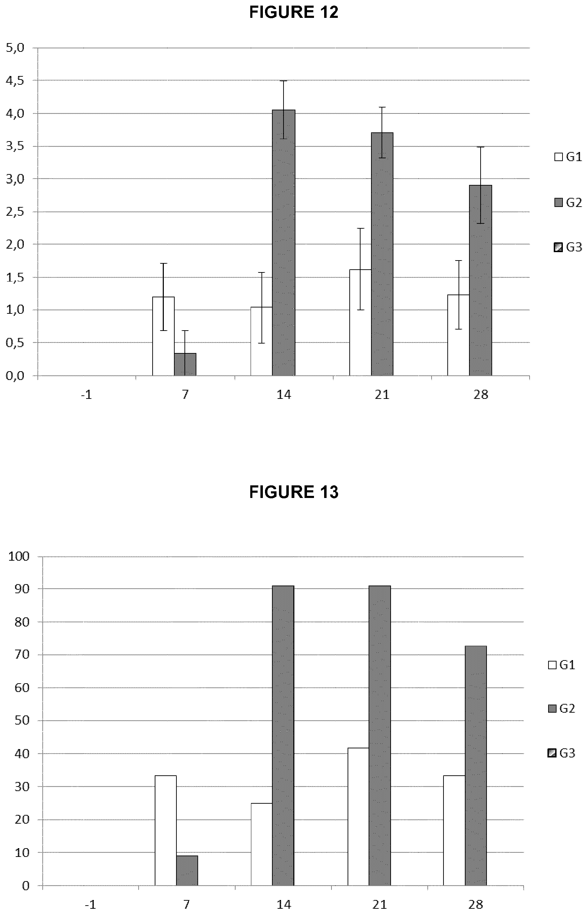

   Figure 12 shows the serum viral load determined in the treatment group 1, and in the control groups 2 and 3 of Example 7.2.
   The day post-infection when the analysis was performed is on the x-axis, and the log₁₀ of the serum viral load of porcine circovirus type 2 (PCV2) expressed in copies/ml determined by quantitative PCR is represented on they-axis.
Figure 13 (not part of the invention)
   Figure 13 shows the percentage of animals showing viremia by PCV2 in the treatment group 1, and in the control groups 2 and 3 of Example 7.2.
   The day post-infection when the analysis was performed is on the x-axis, and the percentage of positive animals is represented on the y-axis.

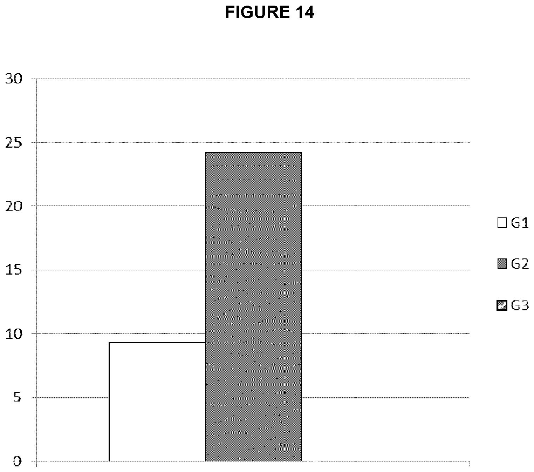

Figure 14 (not part of the invention)
   Figure 14 shows the median surface of affected lung by *Mhyo*-like lesions expressed in percentage for the treatment group 1, and for the control groups 2 and 3 of Example 7.2 at the day 28 post-infection.
   The group is on the x-axis, and the median surface of affected lung by *Mhyo*-like lesions is on the y-axis.

### Detailed Description of the Invention

The present invention provides the following embodiments as defined in items 1-15 below:
1.- A method for preparing a mutant strain of *Mycoplasma hyopneumoniae,* characterized in that it comprises the step of transforming a strain of *M. hyopneumoniae* by means of using a carrier vector comprising at least one exogenous DNA sequence, that sequence being under the control of a DNA sequence of a promoter region of *M. hyopneumoniae,*
   wherein the carrier vector is a replicative plasmid vector comprising:
      1) a DNA sequence comprising the *oriC* region of a strain of *Mycoplasma sp., which is Mycoplasma hyopneumoniae,* and
      2) an exogenous DNA sequence comprising a marker gene, and optionally, an additional exogenous DNA sequence, which are under the control of a DNA sequence of a promoter region of *Mhyo,*
   wherein the promoter region of *Mhyo* corresponds to a DNA segment comprising between 50 base pairs and 300 base pairs located on the 5' side of the gene of a *Mhyo* protein selected from the group formed by the P36, P46, P65, P76, P97, P102, P146, and P216 proteins,
   wherein the promoter region of *Mhyo* initiates or promotes the transcription of a *Mhyo* DNA sequence, and wherein the exogenous DNA sequence is stably introduced in the cytosol of the strain of *Mhyo.*
2.- The method according to item 1, characterized in that the exogenous DNA sequence is selected from the group comprising a resistance gene to an antibiotic, a gene coding for a recombinase, a DNA fragment from *Mhyo,* a gene coding for an antigenic component of a microorganism causing porcine diseases, and combinations thereof.
3.- The method according to any one of items 1 or 2, characterized in that the additional exogenous DNA sequence is selected from the group formed by the DNA sequence coding for the protein of the capsid of the porcine circovirus type 2 (PCV2) and the DNA sequence coding for a protein comprising the protein of the capsid of the porcine circovirus type 2 (PCV2) additionally bearing MetSerGlySer amino acids at the N-terminal end of said protein, said additional exogenous DNA sequence coding for the protein of the PCV2 capsid being selected from the group formed by SEQ ID NO: 3, SEQ ID NO: 4, and SEQ ID NO: 5.
4.- The method according to any one of items 1 to 3, characterized in that the promoter region of *Mhyo* comprises the promoter region of the gene of a *Mhyo* protein selected from the group formed by the P46 and P97 proteins.
5.- The method according to item 4, characterized in that the promoter region of *Mhyo* comprises the promoter region of the gene of the P46 *Mhyo* protein.
6.- The method according to item 4, characterized in that the promoter region of *Mhyo* comprises the promoter region of the gene of the P97 *Mhyo* protein.
7.- A replicative plasmid vector, characterized in that it comprises:
   1) a DNA sequence comprising the *oriC* region of a strain of *Mycoplasma sp., which is Mycoplasma hyopneumoniae,* and
   2) an exogenous DNA sequence comprising a marker gene, and optionally an additional exogenous DNA sequence which are under the control of a DNA sequence of a promoter region of *Mhyo,*

   wherein the promoter region of *Mhyo* corresponds to a DNA segment comprising between 50 base pairs and 300 base pairs located on the 5' side of the gene of a *Mhyo* protein selected from the group formed by the P36, P46, P65, P76, P97, P102, P146, and P216 proteins, and
   wherein the promoter region of *Mhyo* initiates or promotes the transcription of a *Mhyo* DNA sequence.
8.- The replicative plasmid vector according to item 7, characterized in that the additional exogenous DNA sequence is selected from the group formed by the sequence coding for the capsid protein of porcine circovirus type 2 (PCV2) and the sequence coding for a protein comprising the capsid protein of the porcine circovirus type 2 (PCV2) additionally bearing MetSerGlySer amino acids in the N-terminal end of said protein, said DNA sequence coding for the PCV2 capsid protein is selected from the group formed by SEQ ID NO: 3, SEQ ID NO: 4, and SEQ ID NO: 5.
9.- The replicative plasmid vector according to any one of items 7 or 8, characterized in that the promoter region of *Mhyo* comprises the promoter region of the gene of a *Mhyo* protein selected from the group formed by the P46 and P97 proteins.
10.- A mutant strain of *Mhyo,* characterized in that it comprises the replicative plasmid vector according to any one of items 7 to 9.
11.- A vaccine for protecting pigs against porcine enzootic pneumonia caused by *Mhyo,* and optionally against another disease or additional pathological conditions affecting pigs, comprising an immunologically effective amount of the mutant strain of *Mhyo* as defined in item 10.
12.- The vaccine according to item 11, characterized in that the another disease or additional pathological conditions affecting pigs is caused by a microorganism, which is selected from the group formed by *Actinobacillus sp., Brachyspira sp., Pasteurella multocida, Salmonella sp., Streptococcus sp., Isospora sp., Erysipelothrix rhusiopathiae, Leptospira sp., Staphylococcus sp., Haemophilus parasuis, Bordetella bronchiseptica, Clostridium sp., Mycoplasma sp., Lawsonia intracellularis, Escherichia coli,* porcine reproductive and respiratory syndrome virus, swine influenza virus, contagious gastroenteritis virus, porcine parvovirus, encephalomyocarditis virus, coronavirus, rotavirus, porcine circovirus, porcine periweaning failure to thrive syndrome agent, classical swine fever virus, African swine fever virus, calicivirus and torque teno virus (TTV).
13.- The vaccine according to item 12, characterized in that the another disease or additional pathological conditions affecting pigs is caused by porcine circovirus.
14.- The vaccine according to any one of items 11 to 13, characterized in that it further comprises a pharmaceutically acceptable vehicle, and optionally an adjuvant.
15.- A vaccination kit for vaccinating pigs against an infection or disease caused by *Mhyo* and optionally against another disease or pathological conditions caused by microorganisms affecting pigs, comprising a container comprising an immunologically effective amount of the mutant strain as defined in item 10.
Subject-matter disclosed herein pertaining to transposon vectors does not form part of the invention, which is defined by the appended claims.

Disclosed herein is a method for preparing a mutant strain of *Mycoplasma hyopneumoniae* comprising the step of transforming a strain of *M. hyopneumoniae* by means of using a carrier vector comprising at least one exogenous DNA sequence, being that sequence under the control of a DNA sequence having a degree of identity of at least 80% with a promoter region of *M. hyopneumoniae.*

A mutant strain of *Mhyo,* stably incorporating an exogenous DNA sequence in the cytosol and expressing the content of said sequence, can be obtained by the method disclosed herein.

The method may additionally comprise steps that are typical in methods for preparing mutant strains of bacteria, which are well known by the person skilled in the art and that are not essential for said method.

The method thus additionally comprises the steps of:
a) constructing a vector before transferring the vector to the bacterium in the transformation step, and
b) selecting the mutant bacteria that have been transformed with the exogenous DNA sequence after the transformation step.

The efficacy of the method disclosed herein is determined by means of isolating and characterizing the DNA sequences present in the transformed strains, as well as, for example, by means of checking for genotypic or phenotypic characteristics of the mutant strain or by means of checking the production of the recombinant proteins by the same, as it will be described below in the example section.

A mutant strain of *Mhyo,* stably incorporating an exogenous DNA sequence in the cytosol and expressing the content of said sequence, can be obtained by means of the carrier vector used in the method of the invention.

The carrier vector is a replicative plasmid vector.

In the context of the invention "replicative plasmid vector" or "replicative plasmid" is understood as a vector carrying DNA sequences which replicates like an episomal element in the host bacterium, wherein an episomal element is a self-replicating extrachromosomal unit.

In the context of the invention, a replicative plasmid vector is that which allows to incorporate in a stable way an exogenous DNA sequence into the cytosol of a *Mhyo* bacterium and, optionally, to express an RNA or a protein coded by said sequence in the cytosol of the bacterium.

### Exogenous DNA sequence

In the context of the invention, "exogenous DNA sequence" is understood as a DNA fragment that is stably introduced in the cytosol of the strain of *Mhyo.*

It has been found that by means of the method of the invention an exogenous DNA sequence can be stably introduced in a strain of *Mhyo* because said sequence is not lost over successive generations of the bacterium that has been transformed with said exogenous sequence. This stability means that the exogenous DNA sequence can replicate inside the transformed bacterium and in its offspring and can be recovered after multiple generations of the transformed bacterium.

The exogenous DNA sequence can be quite diverse and has a broad definition, as explained below.

In the context of the invention, said sequence can be, for example, a marker gene. The vector comprising the exogenous DNA sequence usually comprises said marker to be able to easily select the mutant strains that have been transformed. Said genetic marker can be, for example, an antibiotic resistance gene. These may include, for example, the *TetM* gene, which confers resistance to the tetracycline from the plasmid pIVT-1, described in Dybvig et al., J. Bacteriol, 2000, 182, 4343-4347, or the *aac(6')-aph(2")* gene, which confers resistance to aminoglycoside antibiotics, such as gentamicin, for example, and which is described in Chow et al., Antimicrob. Agents Chemother., 2001, 45/10), 2691-2694. The gene for phenotypic selection is preferably the *TetM* gene.

The exogenous DNA sequence can also be a gene coding for recombinant proteins such as, for example, the Cre recombinase of bacteriophage P1.

The exogenous DNA sequence can also be a gene coding for a recombinant protein of therapeutic or preventive interest. Said protein can be related to virulence factors or antigenic determinants of microorganisms causing diseases in pigs, and is capable of inducing or contributing to the induction of a protective response against diseases or pathological conditions that may affect pigs. The exogenous DNA sequence preferably codes for a recombinant protein that induces a protective response against diseases or pathological conditions affecting pigs caused by the microorganisms of the group formed by *Actinobacillus sp., Brachyspira sp., Pasteurella multocida, Salmonella sp., Streptococcus sp., Isospora sp., Erysipelothrix rhusiopathiae, Leptospira sp., Staphylococcus sp., Haemophilus parasuis, Bordetella bronchiseptica, Clostridium sp., Mycoplasma sp., Lawsonia intracellularis, Escherichia coli,* porcine reproductive and respiratory syndrome virus, swine influenza virus, contagious gastroenteritis virus, porcine parvovirus, encephalomyocarditis virus, coronavirus, rotavirus, porcine periweaning failure to thrive syndrome agent, classical swine fever virus, African swine fever virus, calicivirus, torque teno virus (TTV) and porcine circovirus. Among them, for example, the gene coding for the glycoprotein 5 (gp5) of the porcine reproductive and respiratory virus (PRRS), the gene coding for the capsid protein of the porcine circovirus type 2 (PCV2), the gene coding for the glycoprotein E (gpE) of the classical swine fever virus, the gene coding for the sp1 protein of the porcine parvovirus (PPV), the gene coding for the toxin of *Pasteurella multocida,* the gene coding for the dermonecrotoxin of *Bordetella bronchiseptica,* the genes coding for the toxins of *Clostridium* spp, *Escherichia coli, or Actinobacillus pleuropneumoniae.* Preferably, said protein induces a protective response against the mentioned diseases.

A DNA fragment from *Mhyo,* optionally reordered or recombined with other DNA fragments, is also considered as an exogenous DNA sequence. Examples of this exogenous DNA sequence are, for example, the origin of replication *oriC* of *Mhyo,* an antisense RNA sequence, or the gene coding for the *Mhyo* P97 or P46 protein, to increase the transcription and translation rate of said gene.

The exogenous DNA sequence is preferably selected from the group comprising an antibiotic resistance gene, a gene coding for a recombinase, a DNA fragment from *Mhyo,* fragment which preferably has been reordered or recombined with one or several DNA fragments, a gene coding for an antigenic component of a microorganism causing porcine diseases, and combinations thereof; more preferably it is a marker gene which confers resistance to an antibiotic, a gene coding for an antigenic component of a microorganism causing porcine diseases, or combinations thereof; and even more preferably it is an antibiotic resistance gene combined with a gene coding for an antigenic component of a microorganism causing porcine diseases, preferably a gene coding for the capsid protein of the porcine circovirus type 2 (PCV2), and optionally combined with a gene coding for a *Mhyo* membrane protein, preferably the *Mhyo* P46 protein.

### Promoter region of Mhvo

In the context of the invention, "promoter region of *Mhyo"* is understood as a DNA sequence characteristic of *Mhyo* which initiates or promotes the transcription of a *Mhyo* DNA sequence.

The DNA sequence which initiates or promotes the transcription of the exogenous DNA sequence has a degree of identity of at least 80%, preferably of at least 85%, more preferably of at least 90%, even more preferably of at least 95%, more preferably of at least 99%, and even more preferably 100% with a promoter region of *Mhyo.*

Preferably, the DNA sequence which initiates or promotes the transcription of the exogenous DNA sequence has a degree of identity of 100% with a promoter region of *Mhyo.*

A sequence, which induces the synthesis of a messenger RNA (transcript) towards its 3' end once identified by the RNA polymerase holoenzyme, is understood as promoter region. This transcript is normally translated and produces a protein with a specific function, or it leads to an RNA that intervenes in protein synthesis or in the regulation of said transcriptional activity.

The skilled person in the art knows the meaning of the expression "a promoter region" and it can be identified by standard means.

The promoter region of *Mhyo* corresponds to a DNA segment preferably comprising at least 50 base pairs, preferably at least 100 base pairs, more preferably at least 200 base pairs, and even more preferably between 200 and 300 base pairs, located on the 5' side of well-established *Mhyo* coding sequences.

As described herein, the DNA sequence used as the promoter in the method and in the carrier vector of the invention can have a degree of identity of 100% with a promoter region of *Mhyo,* comprising at least 50 base pairs.

More specifically, the DNA sequence used as the promoter in the method and in the carrier vector of the invention can have a degree of identity of 100% with a promoter region of *Mhyo,* comprising between 200 and 300 base pairs.

In the context of the invention, "well established coding sequences" is understood as those DNA sequences coding for *Mhyo* proteins or transcriptionally active regions of the *Mhyo* genome which lead to, for example, an RNA that intervenes in protein synthesis or in the regulation of the transcriptional activity. These sequences include, for example, the P46, P65, P97, and P102 surface proteins, described in Minion et al., J. Bacteriol., 2004, 186(21), 7123-7133; the P76, P146, P216 adhesins or the P95 membrane protein, described in Vasconcelos et al., J. Bacteriol., 2005, 187(16), 5568-5577, or the P36 cytosolic protein described in Caron et al., J. Clin. Microbiol., 2000, 38(4), 1390-1396, or ribosomal RNA.

As described herein, the promoter region of *Mhyo can* comprise the promoter region of the gene of a *Mhyo* protein selected from the group formed by the P36, P46, P65, P76, P97, P102, P146, and P216 proteins, more preferably the P46 and P97 proteins, even more preferably it is selected from the group comprising the promoter region of the *mhj_0194* gene of *Mhyo* strain J coding for the P97 protein and corresponding to the DNA sequence comprised between bases 214293 and 214557 of the *Mhyo* strain J (SEQ ID NO: 1), and the promoter region of the gene *mhj_0511* of the *Mhyo* strain J (SEQ ID NO: 2), coding for the P46 protein and corresponding to the DNA sequence comprised between bases 656451 and 656713 of *Mhyo* strain J deposited in the GenBank database with number AE017243 and described in Vasconcelos *et al.,* mentioned above.

The promoter region of *Mhyo,* which controls the exogenous sequences, can be the same for all sequences or can be different. Preferably it is the same.

A DNA promoter sequence can be easily identified by the person skilled in the art by selecting a DNA sequence having a degree of identity of at least 80%, more preferably of at least 90%, more preferably of at least 95%, even more preferably of at least 99%, and even more preferably of 100%, with a promoter region of *Mhyo,* preferably comprising at least 50 base pairs, preferably at least 100 base pairs, more preferably at least 200 base pairs, and even more preferably between 200 and 300 base pairs, located on the 5' side of well-established *Mhyo* coding sequences, and carrying out the method of the invention. The methods for identifying a DNA promoter sequence are described, for example, in the Sambrook and Russell manual, Molecular Cloning 3rd Ed. Cold Spring Harbor Laboratory Press, Cold Spring Harbor New York (2001).

### Replicative plasmid vector

An object of the invention is a replicative plasmid vector comprising:
1) a DNA sequence comprising the *oriC* region of a strain of *Mycoplasma sp.,* and
2) an exogenous DNA sequence comprising a marker gene, and optionally an additional exogenous DNA sequence, which are under the control of a DNA sequence having a degree of identity of at least 80%, 90%, 95%, 99% or 100% with a promoter region of *Mhyo.*

As described herein, the DNA sequence, which initiates or promotes the transcription of the exogenous DNA sequence, can comprise between 200 and 300 base pairs and has a degree of identity of 100% with a promoter region of *Mhyo.*

In the replicative plasmid of the invention, a promoter region of *Mhyo,* selected from the group comprising the promoter region of the gene of the *Mhyo* P97 protein, and the promoter region of the gene of the *Mhyo* P46 protein, is preferably used, the promoter region of *Mhyo* preferably comprises the promoter region of the gene of the *Mhyo* P97 protein, or alternatively the promoter region of the gene of the *Mhyo* P46 protein mentioned above.

The promoter region of *Mhyo,* which controls sequences comprised in the replicative plasmid, can be the same for all the sequences or it can be different. It is preferably the same sequence.

The *oriC* region allows the introduced DNA fragment to replicate like an episomal element and to stably maintain this DNA fragment in the offspring of the transformed cell, preferably maintaining the conditions for phenotypic selection.

In the replicative plasmid vector of the invention the *oriC* region is the *oriC* region of a strain of *Mycoplasma sp.*

In the context of the invention, *Mycoplasma sp.* is understood as any bacterium of the *Mycoplasma* genus, such as, for example, *M. pneumoniae, M. hyopneumoniae, M. hominis, M. genitalium, M. pulmonis, M. capricolum, M. mycoides, M. ovipneumoniae, M. haemofelis, M. gallisepticum, M. laboratorium,* or *M. bovis,* among others.

Preferably, the *oriC* region belongs to a strain of *Mhyo,* more preferably it belongs to strain J, and more preferably it comprises the DNA sequence comprised between bases 897262 and 1802 of *Mhyo* strain J.

The marker gene, also referred to as gene for phenotypic selection, allows selecting the clones which have incorporated the plasmid with the exogenous DNA sequence, as explained above.

Preferably, the vector comprises an additional exogenous DNA sequence. Mutant strains of *Mhyo,* which can be used to prepare monovalent or polyvalent vaccines, optionally marker vaccines, and they can also express useful genetic tools for the subsequent modification of said mutants such as, for example, the Cre recombinase of phage P1, the FLP recombinase of *Saccharomyces cerevisiae,* the tetracycline repressor protein (TetR), or they can completely or partially block the translation of a specific *Mhyo* gene, can be obtained by means of said additional exogenous DNA sequence.

The additional exogenous DNA sequences, which can be incorporated in the replicative plasmid, include, for example, the ORF cre gene coding for the Cre recombinase of phage P1.

Cre recombinase has the capacity to split DNA regions flanked by *loxP* type sequences. Thereby, for example, if the replicative plasmid includes the cre ORF gene, and a resistance gene to an antibiotic flanked by *loxP* type sequences, for example, *lox*66, when Cre recombinase is expressed the antibiotic resistance is eliminated by the transformed bacterium. In other words, in subsequent steps the marker gene can be removed. This is important in the case of preparing transformed mutant strains of *Mhyo* having an attenuated virulence and used for preparing live vaccines, in which the presence of a resistance gene to an antibiotic is an undesirable feature.

Preferably, the replicative plasmid further comprises an additional exogenous DNA sequence coding for a recombinant protein of therapeutic or preventive interest. Said protein can be related to virulence factors or antigenic determinants of microorganisms causing diseases in pigs, and it is capable of inducing or contributing to the induction of a protective response against diseases or pathological conditions which may affect pigs caused by the microorganisms described above.

Preferably, the replicative plasmid comprises at least twice the additional exogenous DNA sequence coding for a recombinant protein of therapeutic or preventive interest, preferably twice, and more preferably twice or three times. In this way several copies of the additional exogenous DNA sequence are introduced and the expression yield of that protein is increased. The promoter region of *Mhyo* which controls the sequences comprised in the replicative plasmid can be the same for all sequences or can be different.

More preferably, the additional exogenous DNA sequence is selected from the group formed by the sequence coding for the capsid protein of porcine circovirus type 2 (PCV2) and the exogenous DNA sequence coding for a protein comprising the capsid protein of porcine circovirus type 2 (PCV2) additionally bearing MetSerGlySer amino acids at the N-terminal end of said protein, wherein said capsid protein is described in the GenBank database with the accession number AAC61864 (SEQ ID NO: 6).

Even more preferably, the additional exogenous DNA sequence codes for the PCV2 capsid protein using the most common codon in the *Mhyo* genome for each of the amino acids corresponding to said protein selected from the group formed by SEQ ID NO: 3, SEQ ID NO: 4, and SEQ ID NO: 5.

SEQ ID NO: 3 has 699 base pairs, SEQ ID NO: 4, has 702 base pairs, and SEQ ID NO: 5 has 992 base pairs, a DNA sequence with a length substantially greater than the other two because it also incorporates the promoter region of the gene of the P97 protein, corresponding to the DNA sequence comprised between bases 214293 and 214557 of the *Mhyo* strain J, to thus simplify the cloning steps. In this description SEQ ID NO: 5 is also referred to as ORF2v2.

Preferably, the additional exogenous DNA sequence comprises the gene of the protein of the PCV2 virus capsid (ORF2) fused to the last base coding for the gene of a *Mhyo* membrane protein, preferably the *Mhyo* membrane protein is selected from the group consisting of the P46 protein and the P97 protein, more preferably it is the P46 protein, more preferably under the control of the promoter of the *Mhyo* P46 or P97 protein genes, and even more preferably under the promoter of the *Mhyo* P46 protein gene.

In the case of the P46 protein, the fusion takes place at the 3' end of the gene coding for said membrane protein (bases 7648-8904). This plasmid directs the synthesis of a hybrid protein which is characterized in that amino acids 1 to 419 are from the P46 protein and amino acids 420 to 652 correspond to the protein of the PCV2 virus capsid, ORF2 version (bases 6946-7647).

Preferably, the additional exogenous DNA sequence comprises the gene coding for the PCV2 capsid protein inserted in the DNA sequence coding for a loop of a *Mhyo* membrane protein, preferably the *Mhyo* membrane protein is selected from the group consisting of the P46 protein and the P97 protein, more preferably it is the P46 protein, more preferably under the control of the promoter of the *Mhyo* P46 or P97 protein genes, and even more preferably under the promoter of the *Mhyo* P46 protein gene.

In the case of the P46 protein, the plasmid directs the synthesis of a hybrid protein which is characterized in that amino acids 1 to 92 are from the N-terminal end of the P46 protein, amino acids 95 to 327 correspond to the protein of the PCV2 virus capsid and amino acids 330-656 are from the C-terminal end of the *Mhyo* P46 protein.

Loops are identified in amino acid sequences by means of methods well known by the person skilled in the art, whereby it is possible to predict their location with reasonable effectiveness.

Said loops generally present high flexibility and have a good tolerance for the insertion of amino acid sequences from other proteins. Furthermore, since they are exposed on the surface of the bacteria, they enable the generation of a good immunogenic response.

Preferably, the replicative plasmid comprises an additional exogenous DNA sequence which is a *Mhyo* gene reversely oriented with respect to the promoter region of *Mhyo,* preferably the *Mhyo* gene codes for a *Mhyo* virulence factor, preferably under the control of the promoter of the *Mhyo* P46 or P97 protein, and even more preferably under the promoter of the *Mhyo* P46 protein.

Said DNA sequence is capable of producing an antisense RNA, which can block the translation of said *Mhyo* gene. If it is a gene coding for a *Mhyo* virulence factor, the transformation may reduce the degree of virulence of the microorganism.

More preferably, the replicative plasmid comprises the sequence comprised between bases 194,535 and 194,654 of the genome of *Mhyo* strain J reversely oriented with respect to the promoter region of *Mhyo.*

Said sequence produces an antisense RNA which can block the translation of the *tlyA* gene of said strain, which corresponds to a *Mhyo* hemolysin.

The Examples describe the preparation of plasmids pOG, pOGCRE, pOGA159, pOGC, pOGL and pOGT. The first incorporates the *aac(6')- aph(2")* gene which confers gentamicin resistance. Furthermore plasmid pOGCRE incorporates the ORF cre gene, coding for the Cre recombinase of phage P1; plasmid pOGA159 incorporates an inhibitory sequence of the translation of the hemolysin a159 gene of *Mhyo;* whereas plasmids pOGC, pOGL and pOGT incorporate furthermore genes coding for different protein versions of the capsid protein of porcine circovirus type 2 (PCV2).

### Transposon vectors (not part of the invention)

Disclosed herein is a transposon vector, specifically a plasmid, comprising:
1) a DNA sequence coding for a transposase,
2) an exogenous DNA sequence comprising a marker gene and,
3) optionally, an additional exogenous DNA sequence,
wherein the DNA sequence coding for a transposase and at least one of the exogenous DNA sequences are under the control of a DNA sequence having a degree of identity of at least 80%, 85%, 90%, 95%, 99% or 100% with a promoter region of *Mhyo.*

The DNA sequence, which initiates or promotes the transcription of the DNA sequences, may comprise between 200 and 300 base pairs and has a degree of identity of 100% with a promoter region of *Mhyo.*

The gene coding for a transposase contains a DNA sequence coding for an enzyme, which binds to a specific sequence in the transposon and catalyzes the movement of said transposon to another part of the genome. The selection of the gene coding for a transposase is not critical. Genes coding for a transposase include transposon *TN4001T* incorporated in plasmid pIVT-1, mentioned above, or transposon Tn916 incorporated in plasmid pAM120, described in, for example, Cao et al., J. Bacteriol., 1994, 176(14), 4459-4462.

The marker gene or gene for phenotypic selection can be a resistance gene to an antibiotic, such as, for example, the *TetM* gene or the *aac(6')-aph(2")* gene, mentioned above. The gene for phenotypic selection is preferably the *TetM* gene.

The marker gene is flanked by two inverted repeats, such as those of the transposon present in the plasmid pIVT-1, mentioned above. Said sequences are recognized by the transposase, and without them the gene for phenotypic selection would not be able to move to the bacterial chromosome and the genetic modification would not be carried out. As the genetic modification is integrated in the genome, it will be stably maintained over the following generations of the transformed cell.

The inverted repeats preferably present the sequences SEQ ID NO: 7 for IRO and SEQ ID NO: 8 for IRI.

A promoter region of *Mhyo* selected from the group comprising the promoter region of the gene of the *Mhyo* P97 protein, and the promoter region of the gene of the *Mhyo* P46 protein, as described above, may be used in the transposon vector. The promoter region of *Mhyo* may comprise the promoter region of the gene of the *Mhyo* P97 protein, or alternatively the promoter region of the gene of the *Mhyo* P46 protein.

The promoter region of *Mhyo,* which controls the sequences comprised in the transposon vector, can be the same for all sequences or can be different. Preferably it is the same.

Similar to that explained in the case of a replicative plasmid, , the transposon vector may further comprise an additional exogenous DNA sequence coding for a recombinant protein of interest. Said protein is capable of inducing or contributing to the induction of a protective response against diseases or pathological conditions which may affect pigs.

The transposon vector may comprise at least twice the additional exogenous DNA sequence coding for a recombinant protein of therapeutic or preventive interest, preferably twice, and more preferably twice or three times. In this way several copies of the additional exogenous DNA sequence are introduced and the expression yield of that protein is increased. The promoter region of *Mhyo,* which controls the sequences comprised in the transposon vector, can be the same for all sequences or can be different.

The additional exogenous DNA sequence may be selected from the sequence coding for the capsid protein of the porcine circovirus (PCV2) and an exogenous DNA sequence coding for a protein comprising the capsid protein of the porcine circovirus (PCV2), additionally bearing MetSerGlySer amino acids at the N-terminal end of said protein, wherein the capsid protein of the porcine circovirus (PCV2) is described in the GenBank database with the accession number AAC61864 (SEQ ID NO: 6).

Preferably, the additional exogenous DNA sequence codes for the PCV2 capsid protein using the most common codon in the *Mhyo* genome for each of the amino acids corresponding to said protein selected from the group formed by SEQ ID NO: 3, SEQ ID NO: 4, and SEQ ID NO: 5, wherein each of the DNA sequences is under the control of a promoter region of *Mhyo.* SEQ ID NO: 5, which already includes the promoter region of the gene coding for the *Mhyo* P97 protein is more preferably used.

Preferably, the additional exogenous DNA sequence comprises the DNA sequence coding for the PCV2 capsid protein fused to the last base coding the gene of a *Mhyo* membrane protein, preferably the membrane protein is selected from the group consisting of the P46 protein and the P97 protein, more preferably it is the P46 protein. The strain transformed with this plasmid expresses the *Mhyo* protein in which the amino acids corresponding to the PCV2 capsid protein are located immediately after its last amino acid, i.e., it expresses a hybrid protein formed by the *Mhyo* P46 or P97 protein and the PCV2 capsid protein. Taking into account that it is a membrane protein, the strain of *Mhyo* transformed with said plasmid expresses the fusion of both proteins in the bacterium membrane. The PCV2 capsid protein is selected from the group formed by SEQ ID NO: 3, SEQ ID NO: 4, and SEQ ID NO: 5, more preferably it is SEQ ID NO: 4. The promoter region of *Mhyo* is preferably the promoter region of the gene coding for the *Mhyo* P97 or P46 protein, and even more preferably the promoter of the *Mhyo* P46 protein.

Preferably, the additional DNA sequence comprises the gene coding for the PCV2 capsid protein inserted into the DNA sequence coding for a loop of a *Mhyo* membrane protein, the membrane protein is preferably selected from the group consisting of the P46 protein and the P97 protein, more preferably it is the P46 protein. The strain transformed with this plasmid expresses the PCV2 capsid protein inserted between the *Mhyo* P46 or P97 protein, i.e., it expresses a hybrid protein formed by the *Mhyo* P46 or P97 protein and the PCV2 capsid protein. The PCV2 capsid protein is selected from the group formed by SEQ ID NO: 3, SEQ ID NO: 4, and SEQ ID NO: 5, more preferably it is SEQ ID NO: 3.

Preferably, the PCV2 capsid protein is inserted between amino acids 92 and 93 of the P46 protein.

The following transposon vectors, in particular plasmids, are especially preferred:
1) Vector comprising the DNA sequence coding for a transposase, preferably transposon *TN4001T,* a DNA sequence coding for a marker gene, preferably the *tetM* gene, optionally flanked by *loxP* type sequences, and a DNA sequence coding for the PCV2 capsid protein, preferably defined by SEQ ID NO: 5, wherein each of the DNA sequences is under the control of a promoter region of *Mhyo* selected between the promoter region of the *Mhyo* P97 protein or P46 protein, preferably the promoter region of the P97 protein. An example of this type of plasmid is that is referred to as pTC3C and described in the Example section.
2) Vector comprising the DNA sequence coding for a transposase, preferably transposon TN*4001T,* a DNA sequence coding for a marker gene, preferably the *tetM* gene, optionally flanked by *loxP* sequences, and a DNA sequence coding for the PCV2 capsid protein, preferably defined by SEQ ID NO: 4, which is fused to the last base coding the gene of a *Mhyo* membrane protein, preferably the membrane protein is selected from the group consisting of the P46 protein and the P97 protein, more preferably it is the P46 protein, wherein each of the DNA sequences is under the control of a promoter region of *Mhyo* selected between the promoter region of the *Mhyo* P97 or P46 protein, preferably the promoter region of the P46 protein. An example of this type of plasmid is that referred to as pTC3T and described in the Example section.
3) Vector comprising the DNA sequence coding for a transposase, preferably transposon TN*4001T,* a DNA sequence coding for a marker gene, preferably the *tetM* gene, optionally flanked by *loxP* type sequences, and a DNA sequence coding for the PCV2 capsid protein, preferably defined by SEQ ID NO: 3, which is fused in a loop of a *Mhyo* membrane protein, preferably the membrane protein is selected from the group consisting of the P46 protein and the P97 protein, more preferably it is the P46 protein, preferably inserted between amino acids 92 and 93 of the P46 protein, wherein each of the DNA sequences is under the control of a promoter region of *Mhyo* selected between the promoter region of the P97 protein or of the *Mhyo* P46 protein, preferably the promoter region of the P46 protein. An example of this type of plasmid is that referred to as pTC3L and described in the Example section.

In these three preferred vectors, the group of exogenous DNA sequences, with the exception of the encoding sequences for the transposase, is flanked by two inverted repeats allowing the incorporation of said group to the genome of the *Mhyo* bacterium to be transformed. The inverted repeats preferably present the sequences SEQ ID NO: 7 for IRO and SEQ ID NO: 8 for IRI.

Another object of the invention is the use of the replicative plasmid vector for preparing a mutant strain of *Mhyo.*

### Strain of Mhyo

Any strain of *Mhyo* can be used in the method of the invention, i.e., the strain can be selected, among others, from field strains, Collection strains (for example, strain J or strain 232) or genetically modified strains.

The strain of *Mhyo* suitable for applying the method of the invention can be isolated from clinical or subclinical cases according to the usual methods used by the person skilled in the art, or it can be selected from the strains which are deposited in Type Culture Collections. In clinical cases, the strain presents the pathology typical of PEP, whereas in subclinical cases the strain is colonizing the animal mucous membranes but does not present any pathology. The strain of *Mhyo* that can be used to apply the method of the invention can show various degrees of virulence. A virulent strain or a strain expressing the main immunogenic determinants of *Mhyo* is preferably used in the method of the invention. The strains that can be used in the method of the invention include strain 232, the genome of which was described in the article by Minion et al., J. Bacteriol., 2004, 186(21), 7123-7133 and collected in the GenBank database with number AE017332; strain J, the genome of which was described in the article by Vasconcelos et al., J. Bacteriol., 2005, 187(16), 5568-5577, and collected in the GenBank database with number AE017243; or a wild type strain such as 6314, which corresponds to a virulent isolate of *Mhyo* belonging to Laboratorios Hipra, S.A. (Amer-Girona-Spain).

### Transformation of the strain

In the context of the invention, the expression "transforming a strain of *Mhyo"* has the usual meaning that is understood by a person skilled in molecular biology and it refers to a method by means of which a bacterium, in this case a *Mhyo* bacterium, incorporates an exogenous DNA sequence and said sequence performs a specific function.

The functions that the exogenous DNA sequence incorporated in the *Mhyo* bacterium can perform include, for example, RNA transcription, protein expression, recombination with other DNA sequences or interruption of the sequence coding for a gene in the genome of the host organism.

The method of transformation is one of the three methods by means of which an exogenous DNA sequence can be introduced in a bacterium. The other two are conjugation, which consists of transferring genetic material between two bacteria in direct contact, and transduction, which consists of injecting an exogenous DNA sequence into the bacterium by means of a bacteriophage virus.

The transformation of a bacterium can be carried out in the method of the invention by means of biochemical treatments known by the person skilled in the art such as, for example, incubation of the bacteria in the presence of a salt containing divalent ions, such as, for example, calcium chloride or calcium phosphate, exposure to a mixture of polyethylene glycol, incubation in the presence of polyvinyl alcohol, use of liposomes in which the exogenous DNA sequence is coated with a lipid, diethylaminoethyldextrane-mediated transfection, or by means of physical treatments such as electroporation, also referred to as electrotransformation, or by biolistics.

In the method of the invention, the bacterium is preferably transformed by incubating the bacteria in the presence of a salt containing divalent ions, exposing it to a mixture of polyethylene glycol, or subjecting it to electroporation; and it is more preferably carried out by electroporation.

Electroporation is a method well known by the person skilled in the art, the experimental protocol of which is described, for example, in the Sambrook and Russell manual, mentioned above.

In the electroporation process, a suspension of the bacteria in an electroporation buffer comprising the carrier vector carrying the exogenous DNA sequence is subjected to an electric pulse causing a change in the structure of the bacterial membrane for the purpose of facilitating the entrance of said sequence into the bacterium.

The electroporation buffer can be, for example, a buffer with pH 7.2 formed by sucrose and 4-(2-hydroxyethyl)-1-piperazineethanesulfonic acid (HEPES), although any buffer suitable for electroporation that is well known by the person skilled in the art can be used, such as those described in the Sambrook and Russell manual mentioned above.

The carrier vector carrying the exogenous DNA sequence is generally used at a concentration comprised between 0.1 and 5 µg/ml, preferably between 0.5 and 2 µg/ml. An electroporation buffer is typically used as a vehicle of said carrier vector.

The suspension of bacteria generally comprises between 10⁶ and 10¹² cfu/ml.

Electroporation can generally be performed at a voltage comprised between 1 and 3 kV, a resistance comprised between 75 and 300 Ω and a capacitance comprised between 10 and 40 µF. In the method of the invention, the electroporation is preferably performed at a voltage comprised between 2 and 3 kV, a resistance comprised between 100 and 175 Ω, and a capacitance comprised between 20 and 30 µF.

Preferably, before performing electroporation, the suspension of *Mhyo* bacteria is subjected to incubation in an electroporation buffer with a divalent ion chelating agent. Although said step is not fundamental in the method of the invention, it has been observed that it significantly increases transformation efficiency.

The chelating agents that can be used for said incubation include, for example, aminocarboxylic acids such as ethylenediaminetetraacetic acid (EDTA), nitrilotriacetic acid (NTA), diethylenetriaminepentaacetic acid (DTPA), or their alkaline salts; hydroxycarboxylic acids such as citric acid, gluconic acid, or their alkaline salts; phosphonic acids, such as 2-aminoethylphosphonic acid, 1-hydroxyethylidene-1,1-diphosphonic acid (HEDP), amino tris(methylenephosphonic acid) (ATMP), ethylenediaminetetra (methylenephosphonic) acid (EDTMP), or their alkaline salts. The chelating agent is preferably EDTA.

Once electroporation is performed, i.e., once the electric pulse is emitted, the suspension of bacteria is preferably incubated with an additional amount of the carrier vector comprising the exogenous DNA sequence.

The same amount of carrier vector that is added before emitting the electric pulse is normally used.

Said incubation is performed by incubating the suspension of bacteria and carrier vector with ice for a time period less than approximately 1 hour, and then keeping it for a time period comprised between 2 and 5 hours at a temperature of 37° C and under a 5% carbon dioxide atmosphere.

More preferably, before performing electroporation, the suspension of *Mhyo* bacteria is subjected to incubation in an electroporation buffer with a divalent ion chelating agent, and after electroporation, the suspension of bacteria is incubated with an additional amount of the carrier vector comprising the exogenous DNA sequence.

As described herein, the transformation can be carried out by using a replicative plasmid vector.

Therefore, the transformation of the strain of *Mhyo* according to the method of the invention allows to obtain a strain of *Mhyo* comprising at least one exogenous DNA sequence in an extrachromosomal vector (or episomal element), which autonomously replicates stably in *Mhyo,* if the transformation has been performed by means of replicative plasmid vectors.

### Obtaining mutant strains

An object of the invention is a mutant strain of *Mhyo* obtainable by the method of the invention.

Another object of the invention is a mutant strain of *Mhyo* comprising at least one exogenous DNA sequence stably incorporated in the cytosol thereof. The exogenous DNA sequence may be controlled by a DNA sequence, which has a degree of identity of at least 80%, preferably of at least 85%, more preferably of at least 90%, even more preferably of at least 95%, more preferably of at least 99%, and even more preferably 100% with a promoter region of *Mhyo.* The exogenous DNA sequence is comprised in the replicative plasmid vector of the invention.

The exogenous DNA sequence may be comprised between IRI and IRO inverted sequence repeats of the transposon vector of the present dlsclosure (not part of the invention).

The mutant strain of *Mhyo* of the invention comprises the replicative plasmid vector of the invention. When a replicative plasmid vector is used, the mutant strain of *Mhyo* comprises said vector in the cytosol thereof. In the case of using a transposon vector, the mutant strain of *Mhyo* incorporates a substantial part of the vector, which is that part comprised between the IRI and IRO inverted sequence repeats, and comprises at least one exogenous DNA sequence.

Another object of the invention is a strain of *Mhyo* transformed by the carrier vector of the invention.

In the context of the invention, "mutant strain of *Mycoplasma hyopneumoniae"* is understood as a strain of *Mhyo* which has been genetically modified by means of using genetic engineering tools.

A mutant strain of *Mhyo* stably incorporating an exogenous DNA sequence, which is functional in said mutant strain, can be obtained by the method of the invention.

In the context of the invention, it is understood that an exogenous DNA sequence is functional in a mutant strain of *Mhyo* when, for example, it expresses the protein coded by said sequence, transcribes RNA or genetically modifies said strain.

In the method of the invention for obtaining transformed strains of *Mhyo,* the starting material is preferably a *Mhyo* exponential growth phase culture.

Said culture is preferably obtained by using a culture medium comprising FriisHS medium (horse serum, yeast extract, heart infusion broth, Hank's balanced salt solution and PPLO (Pleuro-Pneumonia Like Organisms) broth).

The culture is preferably maintained between 48 and 144 hours at a temperature of approximately 37° C under shaking between 100 and 200 rpm until reaching the exponential growth phase.

The bacteria are then isolated, preferably by centrifugation, and subjected to the method of transformation.

After transformation, the suspension of bacteria is preferably spread on plates of FriisHS medium supplemented with agar and the desired selective antibiotic

The transformed colonies showing resistance to the antibiotic of the medium can be recovered after 2 or 3 weeks of incubation at a temperature of 37° C and under a 5% carbon dioxide atmosphere. Said transformed colonies are then preferably inoculated in FriisHS medium supplemented with the selective antibiotic and after about 10 or 20 days the genotype and/or phenotype of the mutants obtained are studied by means of obtaining their genomic DNAs or protein extracts by methods well known by the person skilled in the art.

Transformed mutant strains of *Mhyo* expressing a recombinant protein can be obtained by the method of the invention.

Preferably, the mutant strain of *Mhyo* of the invention expresses the content coded by the exogenous DNA sequence, for example, a protein. The exogenous DNA sequence can be, for example, circovirus (PCV2) capsid protein, or a *Mhyo* protein, for example, the P46 protein, to achieve over-expression thereof. The strains 232Cc6, 6314Cc1, 232Lc2 and 232Tc2, further disclosed herein, incorporate the exogenous DNA sequence in the genome of the bacterium.

Disclosed herein is the mutant strain of *Mhyo* which was deposited by Laboratorios Hipra, S.A. (Amer, Girona, Spain) in Leibniz-Institut DSMZ (Deutsche Sammlung von Mikroorganismen und Zellkulturen GmbH, Inhoffenstraße 7 B, 38124 Braunschweig, Germany) with the accession number DSM 26027 on 29 May 2012. Said strain, referred to as 232Cc6, incorporates the transposon present in plasmid pTC3C on *Mhyo* strain 232. This strain expresses the PCV2 capsid protein in the cytoplasm and in amounts which can be detected by Western blot and ELISA assay.

Disclosed herein is the mutant strain of *Mhyo* which was deposited by Laboratorios Hipra, S.A. (Amer, Girona, Spain) in Leibniz-Institut DSMZ (Deutsche Sammlung von Mikroorganismen und Zellkulturen GmbH, Inhoffenstraße 7 B, 38124 Braunschweig, Germany) with the accession number DSM 26020 on 29 May 2012. Said strain, referred to as 6314Cc1, incorporates the transposon present in plasmid pTC3C on the wild type *Mhyo* strain 6314. This strain expresses the PCV2 capsid protein in the cytoplasm and in amounts which can be detected by Western blot and ELISA assay.

Disclosed herein is the mutant strain of *Mhyo* which was deposited by Laboratorios Hipra S.A. (Amer, Girona, Spain) in Leibniz-Institut DSMZ (Deutsche Sammlung von Mikroorganismen und Zellkulturen GmbH, Inhoffenstraße 7 B, 38124 Braunschweig, Germany) with the accession number DSM 26033 on 29 May 2012. Said strain, referred to as 232Lc2, incorporates the transposon present in plasmid pTC3L on the wild type *Mhyo* strain 232. This strain expresses the PCV2 capsid protein in amounts which can be detected by ELISA assay.

Disclosed herein is the mutant strain of *Mhyo* which was deposited by Laboratorios Hipra, S.A. (Amer, Girona, Spain) in Leibniz-Institut DSMZ (Deutsche Sammlung von Mikroorganismen und Zellkulturen GmbH, Inhoffenstraße 7 B, 38124 Braunschweig, Germany) with the accession number DSM 26034 on 29 May 2012. Said strain, referred to as 232Tc2, incorporates the transposon present in plasmid pTC3T on the wild type *Mhyo* strain 232. This strain expresses the PCV2 capsid protein in amounts which can be detected by ELISA assay.

The following strains also express the protein coded by the exogenous DNA sequence, in that case incorporated in the cytosol of the bacterium as an episomal element:
- the transformed *Mhyo* strain 232POGc9 incorporating the replicative plasmid pOG on wild type *Mhyo* strain 232. Said incorporation has been detected by digesting the total DNA of this strain of *Mhyo* with restriction enzymes and analyzing the resulting fragments by agarose gel electrophoresis.
- the transformed *Mhyo* strain 232POGCREc1 incorporating the replicative plasmid pOGCRE on wild type *Mhyo* strain 232. The expression of the Cre recombinase is detected by means of Western blot and immunodetection.

Mutant strains of *Mhyo,* in which the exogenous DNA sequence incorporated by transposition produces an antisense RNA, which can block the translation of a gene responsible for the virulence of said strain, for example, a gene which corresponds to a *Mhyo* hemolysin, are further disclosed herein.

Preferably, the mutant strain of *Mhyo* incorporates an exogenous DNA sequence in the genome or in the cytosol such that said strain produces an antisense RNA with respect to a gene responsible for the virulence of said strain.

Mutant strains of *Mhyo,* in which the exogenous DNA sequence incorporated by transposition in the genome of the strain interrupts a gene coding for a *Mhyo* virulence factor, are further disclosed herein and they show an attenuated virulence.

Further disclosed herein is a mutant strain of *Mhyo* which incorporates an exogenous DNA sequence which interrupts a gene coding for a *Mhyo* virulence factor, for example a hemolysin. One example is the strain referred to as 232TC3hlyC.

The mutant strains bearing an interrupted gene coding for a *Mhyo* virulence factor can be selected by means of methods well known by the person skilled in the art, such as sequencing the genome of said mutants.

Disclosed herein is the mutant strain of *Mhyo* which was deposited by Laboratorios Hipra, S.A. (Amer, Girona, Spain) in Leibniz-Institut DSMZ (Deutsche Sammlung von Mikroorganismen und Zellkulturen GmbH, Inhoffenstraße 7 B, 38124 Braunschweig, Germany) with the accession number DSM 26049 on 29 May 2012. Said strain, referred to as 232TC3hlyC, incorporates the transposon present in plasmid TC3 on the wild type *Mhyo* strain 232. This strain has an interruption of the ORF coding for the *Mhyo* hlyC virulence factor, so it is a good candidate for being used as an attenuated vaccine strain. The insertion in the genome is detected by means of Southern blot and by direct sequencing.

From the information contained in this description, the person skilled in the art can prepare other transformed strains of *Mhyo* by means of using the method of the invention and by selecting exogenous DNA sequences other than those specifically described.

An object of the invention is the use of a mutant strain of *Mhyo* comprising an exogenous DNA sequence in the cytosol as a host for the expression thereof. The exogenous DNA sequence can code for recombinant proteins or other sequences of interest as explained in this description.

It should be noted that mutant strains of *Mhyo* with very diverse characteristics can be obtained by the method of the invention, so that the person skilled in the art has at his/her disposition a method to genetically modify strains of *Mhyo* and to obtain strains bearing various functionalities.

Examples 5 and 6 show that the mutant strains of *Mhyo* obtained according to the disclosed method surprisingly stably incorporate the exogenous DNA sequence included in the carrier vector and express the content thereof such as, for example, by means of the production of recombinant proteins.

The strain obtained by the method of the invention can be attenuated or inactivated by means of standard procedures well known by the skilled person. In this way it can be used in the preparation of vaccines. Preferably it is used in inactivated form.

By the method of the invention, new strains of *Mhyo* are obtained hitherto undisclosed. Their features allow preparing new vaccines against *Mhyo* and at the same time against other pig pathogens. That is, the mutant strains of the invention allow preparing polyvalent vaccines by using a single strain transformed according to the method of the invention.

### Vaccines

An object of the invention is a vaccine for protecting pigs against porcine enzootic pneumonia caused by *Mhyo,* and optionally against another disease or pathological conditions caused by microorganisms affecting pigs, comprising an immunologically effective amount of the mutant *Mhyo* strain of the invention.

Preferably, the vaccine comprises a pharmaceutically acceptable vehicle and, optionally, an adjuvant agent.

The strain in the vaccine of the invention is inactivated or attenuated, preferably inactivated.

An inactivated vaccine may be obtained by inactivating a pathogenic agent, in that case a bacterium, usually through the use of heat; chemical compounds such as formaldehyde, BEI (binary ethyleneimine) or surfactants; mechanical force using a homogenizer, etc. The inactivation of the pathogenic agent may lead to maintenance of the structure thereof, but being unable to replicate, or to the destruction or disintegration of the structure. In the context of the invention, the term "inactivated vaccine" includes a composition comprising inactivated bacteria, but which retain their structure, as well as a composition in which the structure of the bacteria has been destroyed or disintegrated.

Another object of the invention is the use of the mutant *Mhyo* strain of the invention for preparing a vaccine against porcine enzootic pneumonia caused by *Mhyo,* and optionally against another disease or additional pathological conditions affecting pigs.

The expression "immunologically effective" means that the amount of bacteria administered in the vaccination process is sufficient to induce an effective immunological response in the host against an infection by the virulent forms of *Mhyo* and, in the event that the transformed strain of *Mhyo* expresses a recombinant protein which is capable of inducing or contributing to the induction of a protective response against diseases or pathological conditions which may affect pigs, it is also sufficient to induce an effective immunological response in the host against an infection caused by said microorganism.

The vaccine is preferably aimed at conferring protection to pigs against diseases or pathological conditions such as, for example, those caused by the *Actinobacillus sp., Brachyspira sp., Pasteurella multocida, Salmonella sp., Streptococcus sp., Isospora sp., Erysipelothrix rhusiopathiae, Leptospira sp., Staphylococcus sp., Haemophilus parasuis, Bordetella bronchiseptica, Clostridium sp., Mycoplasma sp., Lawsonia intracellularis, Escherichia coli* microorganisms, porcine reproductive and respiratory syndrome virus, swine influenza virus, contagious gastroenteritis virus, porcine parvovirus, encephalomyocarditis virus, coronavirus, rotavirus, porcine circovirus, porcine periweaning failure to thrive syndrome agent, classical swine fever virus, African swine fever virus, calicivirus and torque teno virus (TTV).

More preferably, the additional disease is that known as PCVAD (Porcine Circovirus Associated Diseases) caused by porcine circovirus (PCV2) whether it presents as a single agent or with the presence of other associated pathogens or microorganisms.

The vaccine of the invention can also be combined, in a single or different recipients, with an additional vaccine or antigenic composition. The additional vaccine or antigenic composition vaccine is preferably aimed at conferring protection to pigs against diseases or pathological conditions such as, for example, those caused by the *Actinobacillus sp., Brachyspira sp., Pasteurella multocida, Salmonella sp., Streptococcus sp., Isospora sp., Erysipelothrix rhusiopathiae, Leptospira sp., Staphylococcus sp., Haemophilus parasuis, Bordetella bronchiseptica, Clostridium sp., Mycoplasma sp., Lawsonia intracellularis, Escherichia coli* microorganisms, porcine reproductive and respiratory syndrome virus, swine influenza virus, contagious gastroenteritis virus, porcine parvovirus, encephalomyocarditis virus, coronavirus, rotavirus, porcine circovirus, porcine periweaning failure to thrive syndrome agent, classical swine fever virus, African swine fever virus, calicivirus and torque teno virus (TTV).

The vaccine of the invention comprises an immunologically effective amount of bacteria of an inactivated or live strain of *Mhyo* which can be obtained according to the method described in the present invention.

It is known that the dose to be used depends on the age and weight of the animal to be vaccinated and on the administration route.

Suitable doses are generally comprised in the range between 10³ and 10¹⁰ color changing units (ccu), preferably between 10⁶ and 10¹⁰ ccu, and more preferably between 10⁸ and 10⁹ ccu.

The animals can be vaccinated at any suitable time. A dose of the vaccine of the invention is preferably administered between 1 and 12 weeks of age, and more preferably the vaccine is administered starting from 3 weeks of age. In specific cases the administration of complementary doses to achieve a satisfactory protection may be necessary. Under these circumstances a second dose is preferably administered between 1 and 5 weeks after the first dose, more preferably after 3 weeks from the first dose. Preferably the vaccine is a single administration vaccine.

The vaccine of the invention is intended for porcine species including, among others, pigs, boars, sows, and piglets of any age or in any phase of their production cycle; it is preferably intended for pigs in the fattening stage, and more preferably for pigs from 1 to 12 weeks of life.

The vaccine can be administered intranasally, intradermally, mucosally or submucosally, subcutaneously, by means of aerosol, intramuscularly, or orally. Preferably it is administered intradermally or intramuscularly.

Said vaccine can be prepared according to the typical methods used by the person skilled in the art for preparing pharmaceutical formulations suitable for the different dosage forms, such as described, for example, in the manual Remington The Science and Practice of Pharmacy, 20th edition, Lippincott Williams & Wilkins, Philadelphia, 2000 [ISBN: 0-683-306472].

The vaccines are typically prepared as injection vaccines in the form of solutions, emulsions or liquid suspensions. They can also be prepared in a solid form suitable to be dissolved or suspended in a liquid vehicle before injection.

The typical volume of a dose of an injection vaccine is between 0.1 ml and 5 ml, preferably between 0.15 ml and 3 ml, and more preferably between 0.2 ml and 2 ml. Usually in the intradermal administration it is used between 0.1 ml and 0.5 ml, preferably between 0.15 ml and 0.4 ml, and more preferably 0.2 ml. In the intramuscular administration, generally it is used between 0.5 ml and 5 ml, preferably between 1 ml and 3 ml, and more preferably between 1 ml and 2 ml.

To immunize an animal with the vaccine of the invention the mutant strain of *Mhyo* is commonly mixed with a pharmaceutically acceptable vehicle.

The liquid vehicles which can be used for preparing the vaccine include, for example, water, saline solution with a physiological salt concentration, or the culture liquid in which the bacteria are cultured.

Additionally, if desired, the vehicle can contain pharmaceutically acceptable excipients or auxiliary substances such as, for example, wetting agents, dispersing agents, emulsifying agents, buffering agents (for example, phosphate buffer), stabilizing agents such as carbohydrates (for example, glucose, sucrose, mannitol, sorbitol, starch, or dextrans), or proteins (for example, albumin, casein, bovine serum, or skimmed milk).

The physicochemical characteristics of the excipients as well as the name of the commercial products under which they are marketed can be found in the book by R.C. Rowe et al., Handbook of Pharmaceutical Excipients, 4th edition, Pharmaceutical Press, London, 2003 [ISBN: 0-85369-472-9].

Adjuvants can also optionally be incorporated in the vaccine to enhance the effectiveness thereof. Preferably the vaccine of the invention further comprises an adjuvant.

Adjuvants are non-specific immune system stimulants which increase immunological response of the host against the invading pathogen. Examples of adjuvants are: aluminium hydroxide, aluminium phosphate, aluminium oxide, vitamin E, squalene, vegetable oil, saponins, ginseng, zymosan, glucans, dimethylaminoethyldextran, dextrans, non-ionic block polymers, polyacrylate (carbomer), complete Freund's adjuvant, incomplete Freund's adjuvant, muramyl dipeptides, W/O, O/W, W/OW type emulsions, and mixtures thereof.

Preferably, the vaccine is an injection vaccine and comprises the mutant strain of the invention inactivated by a non-ionic surfactant. Preferably it is used a non-ionic surfactant selected from the group formed by alkylphenol ethoxylates, ethoxylated sorbitan esters, ethoxylated fatty alcohols, ethoxylated fatty acids, fatty acid alkanolamides, ethoxylated fatty acid alkanolamides, ethoxylated fatty amines, fatty amine oxides, fatty amidoamine oxides, fatty acid glycerides , sucrose esters, alkyl polyglycosides, ethylene oxide and propylene oxide copolymers, and ethoxylated and propoxylated fatty alcohols; more preferably it is selected from alkylphenol ethoxylates and ethoxylated sorbitan esters, and yet more preferably it is an alkylphenol ethoxylate, such as Triton^{®} X-100 marketed by Dow, for example.

More preferably, the vaccine is an injection vaccine and comprises the mutant strain of the invention inactivated by a non-ionic surfactant and as an adjuvant, a W/O/W type emulsion, such as the product Montanide^{®} ISA 201 marketed by the company SEPPIC (France), for example.

Preferably, the vaccine can comprise the strain of the invention in a lyophilized form. The lyophilization process is carried out by means of methods well known by the person skilled in the art.

### Vaccination kit

The object of the present invention also includes a vaccination kit for vaccinating pigs against an infection or a disease caused by *Mhyo* and optionally against another disease or pathological conditions caused by microorganisms affecting pigs.

Said vaccination kit comprises a container comprising an immunologically effective amount of the mutant strain of the invention or a vaccine of the invention.

The vaccination kit disclosed herein can comprise a combination of the vaccine of the invention with an additional antigenic composition, which are contained in a single recipient or in different recipients. Such additional antigenic composition is aimed at conferring protection to pigs against diseases or pathological conditions such as those mentioned in the Vaccines section.

The industrial application of the invention is clearly inferred from the description. It should be pointed out that PEP is a widespread global chronic respiratory disease responsible for great economic losses in the pig industry and that the possibility of genetically modifying strains of *Mhyo* in a targeted and stably manner allows the preparation of vaccines with superior properties than those of conventional bacterins. Attenuated mutant strains of *Mhyo,* mutant strains over-expressing a specific virulence factor, strains inhibiting a specific virulence factor, or strains further expressing antigenic components of microorganisms responsible for diseases affecting pigs, for example, which are suitable for preparing effective vaccines against PEP and against other porcine pathologies, can be prepared by means of the method of the invention.

The targeted genetically modification of *Mhyo* in stable form for the first time, has allowed the inventors, inter alia, the use of these transformed strains as expression vector of exogenous DNA sequences like, for example, nucleotide sequences coding for proteins of therapeutic or preventive interest such as the protein of the PCV2 capsid, among others. By means of the technology disclosed in this invention, new vaccine polyvalent candidates have been designed and prepared, which can be used to prevent different diseases at the same time by administering a single strain.

### Assay for the production of recombinant proteins bv transformed strains of Mhyo according to the method of the invention

The ELISA method can be used to identify the presence of the protein expressed by the exogenous DNA sequence in the mutant strains of *Mhyo* of the invention.

In particular, to detect the presence of the PCV2 capsid protein and to quantify it in the strains of *Mhyo* obtained, a commercial kit, which allows detecting and estimating in an approximate manner the amount of said antigen by means of plate immunodetection (ELISA), was used.

The protein extracts were obtained after concentrating them in exponential-phase cultures of each strain and lysing the cells obtained.

The results were positive indicating that the proteins coded by ORF2 or ORF2v2 are expressed at a greater or lesser amount in all the assayed strains and plasmids (Figure 5B).

The strains with the version originating from plasmid pTC3C were also analyzed by means of denaturing polyacrylamide gel electrophoresis and Western blot (Figure 5C). Bands of the same molecular weight as the PCV2 capsid protein were detected.

It can be observed that proteolytic degradation bands, a common problem in recombinant protein expression, did not appear in the electrophoresis gels.

To that end, the PCV2 capsid protein expressed in a recombinant manner in *Mhyo* can be a good antigen for formulating a polyvalent vaccine. Likewise, *Mhyo* is a good host for the recombinant production of proteins.

### Vaccination assays

The effectiveness of the vaccines containing the mutant strains of *Mhyo* disclosed herein was tested, wherein said strains comprised the exogenous DNA sequence coding for the PCV2 capsid protein in the genome of the bacteria, and they expressed said protein in the cytosol of the bacteria.

To that end, vaccines containing strains 6314Cc1 or 232Cc6 were tested, and the animals were vaccinated with a single dose or were revaccinated with a second dose, as described in the Examples section.

Additionally, two groups of animals were incorporated in the study: a non-vaccinated, but infected group (group 5), and a non-vaccinated and non-infected group (group 6).

The following responses were assessed: the immunological response against *Mhyo,* the immunological response against PCV2, and the PCV2 viral DNA load in the serum of the animals.

By observing the results of the vaccination assays performed with the vaccines comprising mutant strains of *Mhyo* obtained according to the method shown in Figures 6, 7 and 8, it can be concluded that the transformed strains of *Mhyo* expressing the PCV2 capsid protein generate a significant immunological response against *Mhyo* and, surprisingly, also against PCV2, significantly reducing PCV2 viral load such that they can be used in vaccines against infections caused by *Mhyo* and by PCV2 at the same time.

A vaccine containing a mutant strain of *Mhyo* disclosed herein was also tested against an infection by PCV2 and *Mhyo,* wherein such strain comprised the exogenous DNA sequence coding for the PCV2 capsid protein in the genome of the bacterium, and expressed such protein in the cytosol of the bacterium.

It was tested a vaccine containing the 6314Cc1 strain, animals were vaccinated with a single dose and were challenged with a PCV2 isolate and with a pathogenic strain of *Mhyo,* as described in the Examples.

Additionally, two groups of animals were incorporated in the assay: a non-vaccinated but infected (group 2), and a non-vaccinated and non-infected (group 3).

The responses assessed were: the immunological response against *Mhyo,* the immunological response against PCV2, the PCV2 viral DNA load in the serum of animals, and the *Mhyo*-like lesions in the lungs of animals.

It was observed that the strain of *Mhyo,* genetically modified according to a method disclosed herein, which expresses the capsid protein of PCV2, generates a significant reduction of the PCV2 viral DNA load and of the *Mhyo*-like lesions in the lungs of animals compared with the non-vaccinated animals.

A vaccine containing a mutant strain of *Mhyo* obtained according the method of the invention was also tested, wherein such strain comprised a replicative plasmid vector comprising the exogenous DNA sequence coding for the PCV2 capsid protein and expressed such protein in the cytosol of the bacterium. As in the previous tests, it was observed that the strain of *Mhyo,* genetically modified according to the method of the invention by means of replicative plasmids, which expresses the PCV2 capsid protein, generates a significant reduction in the PCV2 viral load.

### Testing the degree of attenuation of mutant strains

The mutants obtained disclosed herein include strain 232TC3hlyC containing the plasmid pTC3 incorporated by transposition in the region of the genome coding for a *Mhyo* virulence factor: hemolysin C, coded by the *hlyC* gene.

To assess the attenuation degree of said strain, 8 week-old pigs were selected and divided into two groups of 8 animals per group. The first group was intratracheally infected with a dose of strain 232TC3hlyC, and the second group with a dose of the wild type strain of *Mhyo, Mhyo* strain 232, before being modified.

The animals were euthanized 28 days after the infection and it was observed that no colonization occurred in the nasal area in any of the two groups of animals, whereas 25% of the animals from the group infected by the wild type strain showed bronchial colonization.

None of the animals infected with the transformed strain of *Mhyo* showed bronchial colonization.

Therefore, the transformed *Mhyo* strain 232TC3hlyC shows attenuation in relation to the wild type parental strain and it can be used, as such, as a vaccination candidate in an attenuated live vaccine.

The *Mhyo* mutant strain 6314POGAc4 was obtained by the method of the invention. Said mutant strain inhibits the expression of *Mhyo* hemolysin 159 gene, since the exogenous DNA sequence incorporated by means of a replicative plasmid (pOGA159) produced an antisense RNA corresponding to a gene coding for a *Mhyo* hemolysin. When the animals were infected with this strain, it was observed that the strain showed attenuation with respect to the wild type parental strain. Said mutant strain was characterized by a reduced ability to colonize the upper and lower respiratory tract, as well as by a reduced capacity to cause lesions typical of PEP.

The following Examples 1, 2, 4.6 to 4.8, 5 and 8.2 are set out to provide the person skilled in the art with a sufficiently clear and complete explanation of the present invention, but they must not be considered as limiting the essential aspects of the object thereof set out in the preceding sections of this description.

### Examples

The recombinant DNA techniques and methods applied below are described in detail in the manuals by Sambrook and Russell, Molecular Cloning 3rd Ed. Cold Spring Harbor Laboratory Press, Cold Spring Harbor New York 5 (2001) and by Ausubel et al., Current Protocols In Molecular Biology, John Wiley and Sons, Inc. (1998).

*E. coli* XL1-blue strain (Stratagene) has been used as the host for the vectors based on plasmids pMTn4001 or pBluescript II SK.

Unless otherwise indicated, all the DNA sequences described in the plasmid constructs described in this section come from the sequence deposited in the GenBank database with number AE017243. This genomic sequence corresponds to that of *Mhyo* strain J described by Vasconcelos et al., J. Bacteriol., 2005, 187(16), 5568-5577.

All the oligonucleotide sequences described below are written in 5' to 3' direction unless otherwise indicated. Phusion thermopolymerase (Finnzymes) having proof reading activity was used in all PCR reactions.

The underlined oligonucleotide sequences indicate the presence of a restriction enzyme site which is specified in the name of each of the sequences.

### Example 1.- Choosing a strain of Mhyo

The wild type strains of *Mhyo* used were strain 6314 corresponding to a virulent *Mhyo* isolate from Laboratorios Hipra S.A. (Amer-Girona-Spain) and Strain 232 from Iowa State University (Ames-Iowa-USA).

### Example 2.- Construction of replicative plasmids

### Example 2.1.- Construction of replicative plasmid pOG

To obtain a replicative plasmid in *Mhyo,* the *oriC* region of *Mhyo* amplified by means of PCR was introduced in a plasmid pBluescript II SK. The oligonucleotides 5OriCNotI (ATG CGC GGC CGC TTA TTT ATC AGA AAC AGT TAG) (SEQ ID NO: 9) and 3OriCXbaI (AGT CGG GCC CAG CTT GCG CAT CAT TGG ATG ATG GAT TC) (SEQ ID NO: 10) were used for this amplification and the genomic DNA of *Mhyo* strain J was used as a template. The 1.96 kb fragment obtained was then digested with restriction enzymes *Not*I and *Xba*I. On the other hand, the gentamicin resistance gene (*aac(6')-aph(2")* was amplified by PCR by means of oligonucleotides 5GmORF (ACT GGG ATC CAT GAA TAT AGT TGA AA ATG) (SEQ ID NO: 11) and 3GmApa (GGA TGG GCC CAG CTT GCG CAT CAT TGG) (SEQ ID NO: 12) and using plasmid pIV-T as a template. The 1.8 kb PCR product was then digested with the corresponding restriction enzymes. The promoter region of the gene of the P97 protein was amplified by PCR by means of oligonucleotides 5'p97*Xba*I (G ATC TCT AGA TCG AGG AAG ACT GAT TAG AAA TTT AGA ACT) (SEQ ID NO: 13) and 3'p97BamHI (GAT CGG ATC CAC TCA TAT TTT AAA CCT CAA TTA T) (SEQ ID NO: 14) using DNA of *Mhyo* strain J as the template. The PCR product (266 bp) was then digested with the corresponding restriction enzymes. Finally, the three fragments obtained were ligated in plasmid pBluescript II SK previously digested with the restriction enzymes *Xba*I and *BamH*I and the ligation mixture was transformed into *Escherichia coli* XL1-blue cells. Colonies bearing the desired construct were identified from the colonies resulting from the transformation by means of restriction pattern analysis and sequencing. One of the positive clones was named pOG and selected for the next steps (Figure 1).

### Example 2.2.- Construction of replicative plasmid pOGCRE

Replicative plasmid pOGCRE was obtained by digesting plasmid pOG with restriction enzymes *Bam*HI and *Apa*I and recovering a 5.1 kb fragment. On the other hand, the gentamicin resistance gene (*aac(6')*- *aph(2")* was amplified by means of PCR from plasmid pIV-T using oligonucleotides 5GmORF and 3GmORFSpel (ACT GAC TAG TAG CTT GCG CAT CAT TGG) (SEQ ID NO: 15) a 1.8 kb fragment which was then digested with restriction enzymes *Bam*HI and *Spe*I being obtained. The gene corresponding to Cre recombinase was amplified by means of PCR using plasmid pSH62 (Euroscarf) and oligonucleotides 5CreBgIII (ATG CAG ATC TAT GTC CAA TTT ACT GAC CGT ACA CCA AAA TTT G) (SEQ ID NO: 16) and 3CreApal (ATG CGG GCC CTTA ATC GCC ATC TTC CAG CAG GCG CAC) (SEQ ID NO: 17) as the template. The PCR product (1.0 kb) was cleaved with the enzymes *BgI*II and *Apa*I. Lastly, the plasmid pOG was digested again, this time with restriction enzymes *Bam*HI and *Xba*I, and it was recovered a band of 266 bp corresponding to the promoter of the gene of the P97 protein. The four fragments obtained were ligated by means of T4 DNA ligase and the ligation reaction was transformed into *E*. *coli* XL1-blue cells. From the resulting transformation colonies the ones bearing the desired construct were identified by means of restriction patern analysis. One of the positive clones named pOGCRE was selected for the next steps (Figure 1).

### Example 2.3.- Construction of replicative plasmid pOGC

To obtain plasmid pOGC, plasmid pBluescript II SK was digested with the enzyme *Not*I, and once purified it was dephosphorylated with alkaline phosphatase enzyme. At the same time, a 992 bp fragment was obtained by digesting the synthetic gene ORF2v2 (SEQ ID NO: 5) with restriction enzymes *Spe*I and *Not*I. Using plasmid pOGCRE as a template, a DNA segment was amplified with oligonucleotides 3gmORFSpel and 5OriCNotI by means of PCR and once purified, the PCR product was digested with enzymes *Spe*I and *Not*I. The three fragments obtained were ligated by means of T4 DNA ligase and the ligation reaction was transformed into *E. coli* XL1-blue cells. The positive clones were identified by means of restriction mapping and sequencing (Figure 2).

### Example 2.4.- Construction of replicative plasmid pOGL

To obtain plasmid pOGL, plasmid pOGC was digested with enzymes *Not*I and *Spe*I and bands of 2.8 kb and 4 kb were recovered. The DNA fragment corresponding to the band of 2.8 kb was dephosphorylated with alkaline phosphatase. At the same time, a 2.25 kb fragment was obtained by digesting the plasmid pTC3L (see Example 3.4) with restriction enzymes Spel and *Not*I. The three fragments obtained were ligated by means of T4 DNA ligase and the ligation reaction was transformed into *E*. *coli* XL1-blue cells. The positive clones were identified by means of restriction mapping and sequencing (Figure 2).

### Example 2.5.- Construction of replicative plasmid pOGT

To obtain plasmid pOGT, plasmid pOGC was digested with enzymes *Not*I and *Spe*I and bands of 2.8 kb and 4 kb were recovered. The DNA fragment corresponding to the band of 2.8 kb was dephosphorylated with alkaline phosphatase. At the same time, a 2.23 kb fragment was obtained from the digestion of plasmid pTC3T (see Example 3.5) with restriction enzymes *Spe*I and *Not*I. The three fragments obtained were ligated by means of T4 DNA ligase and the ligation reaction was transformed into *E. coli* XL1-blue cells. The positive clones were identified by means of restriction mapping and sequencing (Figure 2).

### Example 2.6.- Construction of replicative plasmid pOGA159

Replicative plasmid pOGA159 was constructed in two consecutive cloning steps. By means of a PCR reaction with oligonucleotides 5'p97ApaI and 3TetMClaI (TGT TAT CGA TACC GTC GAT GCA CCT CGA GCT AAG TTA TTT TAT TG) (SEQ ID NO: 18) and using plasmid pMTn4001 as a template, a 2.2 kb fragment corresponding to the promoter of the gene of P97 protein was amplified. This PCR product was digested with restriction enzymes *Cl*aI and *Apa*I. On the other hand, and using plasmid pOG as a template, a 1.9 kb fragment was amplified with oligonucleotides 5OriCClaI (AGA CAT CGA AAG CTT GAT TAT GCT GAT TGC ATT CTT TCA ATT TG) (SEQ ID NO: 19) and 5OriCEcoRI (AGA GGA AT CC GAT TTA TTT ATC AGA AAC AGT TAG TCT TTT CC) (SEQ ID NO: 20) corresponding to the *Mhyo oriC.* This PCR product was digested with restriction enzymes *Eco*RI and *Cl*aI. Subsequently, by means of PCR a 316 bp fragment corresponding to the promoter of P97 protein gene was amplified with oligonucleotides 5Xbalpp97 (AGA CTC TAG AAC TAG TGG ATC CCC CGG GCC CCT CGA GGA AGA CTG ATT AGA AAT TTA G) (SEQ ID NO: 21) and 3EcoRIpp97 (AGA CGA ATT CGA ATT CCT GCA GGG ATC CAC TCA TAT TTT AAA CCT C) (SEQ ID NO: 22). Once purified, this fragment was digested with enzymes *Eco*RI and *Bam*HI. The last step of this first cloning process was digest plasmid pBluescript II SK with restriction enzymes *Xba*I and *Apa*I. These four DNA fragments were ligated by means of T4 DNA ligase and the ligation reaction was transformed into *E. coli* XL1-blue cells. Colonies bearing the desired construct were identified from the colonies resulting from the transformation by means of restriction pattern analysis. The new plasmid thus obtained was digested with restriction enzyme *Eco*RI. Lastly the DNA designed for silencing was amplified by PCR with oligonucleotides 5ahlycEcoRI (CAT CGA ATT CAC GAA TTA GTG ATT CTG CCT TTT C) (SEQ ID NO: 23) and 3ahlycEcoRI (CAT CGA ATT CAT TAA AGT TGA TTC GGT GTT TAA TC) (SEQ ID NO: 24). Once digested with restriction enzyme *Eco*RI and dephosphorylated by means of alkaline phosphatase, DNA fragments obtained were ligated by means of T4 DNA ligase and the ligation reaction was transformed into *E*. *coli* XL1-blue cells. Colonies bearing the desired construct were identified from colonies resulting from the transformation by means of sequencing (Figure 1).

### Example 3.- Construction of plasmids for transformation by transposition Example 3.1.- Construction of plasmid pTC3 for transformation by transposition (not part of the invention)

Plasmid pMTn4001 (Pich et al., Microbiology 152:519-527 (2006)) was amplified by PCR with oligonucleotides 5pMTn4001Pstl (CAT GCT GCA GCC CGG GGG ATC CAC TAG TTC TAG AG) (SEQ ID NO: 25) and 3pMTn4001 (GTA CCC AAT TCG CCC TAT AGT GAG TCG) (SEQ ID NO: 26). Oligo 3pMTn4001 was phosphorylated at its 5' end. The product of this PCR reaction (2.98 kb) was digested with restriction enzyme *PstI.* On the other hand, the promoter of the gene of P97 protein was amplified by means of PCR with oligonucleotides 5'p97 (TCG AGG AAG ACT GAT TAG AAA TTT AGA ACT) (SEQ ID NO: 27) and 3'p97BamHI using the DNA of *Mhyo* Strain J as a template. This PCR product (280 bp) was digested with restriction enzyme *BamHI* and dephosphorylated with alkaline phosphatase. The same promoter was also amplified by means of a second pair of oligonucleotides: 5'p97Apal (GAT CGG GCC CTC GAG GAA GAC TGA TTA GAA ATT TAG AAC T) (SEQ ID NO: 28) and 3'p97BamHI and digested with restriction enzymes *ApaI* and *BamHI.* The transposase gene was amplified by means of PCR using plasmid pMTn4001 as a template and oligonucleotides 5'TnpBamHI (GAT CGG ATC CAT GAC CCA AGT ACA TTT TAC ACT GAA AAG) (SEQ ID NO: 29) and 3'TnpApal (GAT CCT CGA GGG GGG GCC CTT TTA CAC AAT TAT ACG GAC) (SEQ ID NO: 30). The obtained band of 978 bp was then digested with restriction enzymes *BamHI* and *ApaI.* Lastly, the fragment corresponding to tetracycline resistance *tetM* was amplified from plasmid pMTnTetM438 (Pich et al., Microbiology 152:519-527 (2006)) using the oligonucleotides 5'TetMBamHI (GAT CGG ATC CAT GAA AAT TAT TAA TAT TGG AGT T) (SEQ ID NO: 31) and 3' TetMPstI (GAT CGC TGC AGG AAT TCG ATA TCA AGC TTA TCG ATA CCG TCG ATG CAC CT) (SEQ ID NO: 32). The 1.9 kb fragment obtained was digested with restriction enzymes *SalI* and *BamHI* and dephosphorylated with alkaline phosphatase. The five resulting fragments were ligated by means of T4 DNA ligase and the ligation reaction was transformed into *E. coli* XL1-blue cells. From the resulting transformation colonies the ones bearing the desired construct were identified by means of restriction patern analysis and sequencing. One of the positive clones named pTC3 was selected for the next steps (Figure 3).

### Example 3.2.- Construction of plasmid pTC366 for transformation by transposition (not part of the invention)

This plasmid is directly derived from pTC3 by the inclusion of two 34 bp lox66 sequences (ATA ACT TCG TAT AGC ATA CAT TAT ACG AAC GGT A) (SEQ ID NO: 33) flanking the tetracycline resistance gene. To that end, the tetracycline resistance gene sequence TetMp97 of plasmid TC3 was amplified by PCR with oligonucleotides 5loxp66Tetp97 (GAT TAA GGG CCC ATA ACT TCG TAT AGG ATA CTT TAT ACG AAG TTA TGT CGA CCC CCT CGA GGA AGA CTG) (SEQ ID NO: 34) and 3loxp66Tetp97 (GGG ACT AGT TAC GGT TCG TAT AAT GTA TGC TAT ACG AAG TTA TCT GCA GGA TAT CAA GCT TAT CGA TAC CGT CG) (SEQ ID NO: 35), obtaining a 2.2 kb PCR fragment which was digested with restriction enzymes *Ap*aI and *Spe*I. At the same time, plasmid TC3 was digested with the same restriction enzymes and the band of 4.4 kb was recovered. The two fragments were ligated using T4 DNA ligase and the ligation mixture was transformed into *E. coli* XL1-blue cells. A positive clone, which had correctly incorporated *lox*66 sequences and which was called pTC366 (Figure 3), was identified by sequencing from the colonies resulting from the transformation.

### Example 3.3.- Construction of plasmid pTC3C for transformation by transposition (not part of the invention)

To obtain plasmid pTC3C, plasmid pTC366 was digested with enzymes *Spe*I and *Not*I. A 992 bp fragment originating from the digestion of synthetic gene ORF2v2 (SEQ ID NO: 5) with the same restriction enzymes was cloned on this plasmid. The positive clones were identified by means of restriction mapping (Figure 3).

### Example 3.4.- Construction of plasmid pTC3L for transformation by transposition (not part of the invention)

By means of a PCR reaction with oligonucleotides P46CtFdHindlll (GTG TAA GCT TAA AAA CCA GGA TGC ACA AAA TAA C) (SEQ ID NO: 36) and P46CtRv-Notl (TGT TGC GGC CGC TTT AGG CAT CAG GAT TAT CAA C) (SEQ ID NO: 37) and using the genomic DNA of strain 232 as a template, a 1.0 kb fragment corresponding to the 3' end of the gene of P46 protein was amplified. This PCR product was digested with restriction enzymes *Hind*III and *Not*I. On the other hand, and by using the same genomic DNA as a template, a 276 bp fragment was amplified with oligos P46NtFdSpel (AGA CAC TAG TTT GAA TTT GTA TTT TCC ATA ATC) (SEQ ID NO: 38) and P46NtRvBamHI (AGA GGG ATC CTG TAA TTG TTG AAG TTG CTG CCT) (SEQ ID NO: 39). This fragment corresponding to the promoter and to the 5' end of P46 protein gene was then digested with restriction enzymes *Spe*I and *Bam*HI. Lastly, and by using as a template plasmid pTC3C together with oligonucleotides CircRv-NotI (TGT TGC GGC CGC TTA TGG TTC TAA 55 TGG TGG ATC) (SEQ ID NO: 40) and Circ2Fd-BamHI (GTG TGG ATC CAT GAC ATA TCC AAG AAG AAG AT) (SEQ ID NO: 41), the gene corresponding to PCV2 capsid protein was amplified. This 712 bp DNA fragment was then digested with restriction enzymes *Not*I and *Bam*HI. All the fragments obtained were cloned in plasmid pTC366 previously digested with enzymes *Spe*I and *Not*I. The positive clones were identified by means of restriction mapping (Figure 3).

### Example 3.5.- Construction of plasmid pTC3T for transformation by transposition (not part of the invention)

In this example, oligonucleotides P46NtFdSpel and P46CIRCRv (ATC TTC TTC TTG GAT ATG TCA TGG CAT CAG GAT TAT CAA CAT TA) (SEQ ID NO: 42) were used for the PCR amplification of the promoter region and the 5' end of the coding region of the gene of P46 protein starting from the genomic DNA of strain 232. The band of 1.25 kb obtained was cleaved with restriction enzyme *Spe*I. At the same time a second PCR reaction was performed with oligonucleotides P46CIRCFd (TAA TGT TGA TAA TCC TGA TGC CAT GAC ATA TCC AAG AAG AAG AT) (SEQ ID NO: 43) and CircRv-Notl on pTC3C obtaining a 732 bp DNA fragment. By means of a recombinant PCR reaction in which the preceding fragments were used as a template and using the oligonucleotides P46NtFdSpel and CircRv-Notl, a 2.2 kb fragment was obtained. This fragment was digested with restriction enzymes Spel and *Not*I and ligated with plasmid pTC366 previously digested with enzymes Spel and *Not*I. The positive clones were identified by means of restriction mapping and sequencing (Figure 3)

### Example 4.- Obtaining transformant bacteria

All the products and reagents used in this method were sterilized by means of autoclaving, filtration or irradiation. The starting material was a *Mhyo* culture in exponential growth phase. This growth phase is preferably obtained by using a 1/100 dilution of the inoculum and leaving the culture to grow for about 48-144 hours in FriisHS medium (20% (v/v) irradiated horse serum, 2.4 % (v/v) yeast extract, 0.1% (v/v) phenol red, 0.4% "Hanks' Balanced salt solution", 0.058% (w/v) "Heart Broth Infusion", 0.054% (w/v) PPLO and ampicillin at a final concentration of 100 µg/ml). Once about 40 ml of *Mhyo* culture in stationary phase were obtained, the content of the culture flasks was pipetted 10 times to separate the cells and the culture was then filtered with a 0.45 µm filter to finish separating the cells. The cells were then centrifuged for 20 minutes at 20,000xg, resuspended in an electroporation buffer (272 mM Sucrose, 200 mM Hepes pH 7.2) supplemented with 1 mM EDTA and were incubated in ice for 10 minutes. After 10 minutes of incubation with EDTA the centrifugation was repeated at 20,000xg for 20 minutes and the cells were resuspended in a volume between 100 and 300 µl of electroporation buffer. 20 µg of plasmid previously dissolved in electroporation buffer at a concentration between 0.5 and 2 µg.ml⁻¹ and the cell suspension up to a final volume of 100 µl were added into a 0.2 cm electroporation cuvette. When performing the electroporation, the variables of the electroporation machine were set to a voltage of 2.5 kV, resistance between 100 and 175 Ω and capacitance of 25 µF. The typical "time constant" values obtained were between 2.3 and 3 ms. Once the electric pulse was emitted, 900 µl of FriisFBS medium (exactly the same formulation as the FriisHS medium, but replacing the horse serum with fetal bovine serum) and additional 20 µg of plasmid were added. The resulting suspension was incubated for 20 minutes on ice and then 3 hours at 37° C and 5% CO₂. After 3 hours, the suspension obtained was distributed (about 200 µl per plate) on FriisHS plates supplemented with 0.7% (w/v) low melting point agar and the desired selective antibiotic (0.5 µg/ml tetracycline and/or 10 µg/ml gentamicin).

After 2 or 3 weeks of incubation at 37° C and 5% CO2, the transformant colonies in the Friis HS plates were recovered and inoculated in 50 ml flasks containing 10 ml of FriisHS medium supplemented with the selective antibiotic at the previously indicated concentrations. Once the medium changed its color (between 10 and 20 days after incubation at 37° C and 150 rpm) genotypic and/or phenotypic studies of the obtained mutants were conducted by obtaining genomic DNAs or protein extracts as it is known by any person skilled in the art.

### Examples 4.1. to 4.8. - Obtaining transformed mutant strains of Mhyo (Examples 4.1 to 4.5 are not part of the invention)

By substantially following the method described in Example 4, the transformed mutant strains of *Mhyo* shown in Table I were prepared using the plasmids which are also listed in the same table.

**Table I**

| Example | Plasmid | Parental strain | Transformed mutant strain |
|---|---|---|---|
| 4.1 | pTC3C | 232 | 232Cc6 |
| 4.2 | pTC3C | 6314 | 6314Cc1 |
| 4.3 | pTC3L | 232 | 232Lc2 |
| 4.4 | pTC3T | 232 | 232Tc2 |
| 4.5 | pTC3 | 232 | 232TC3hlyC |
| 4.6 | pOG | 232 | 232POGc9 |
| 4.7 | pOGCRE | 232 | 232POGCREc1 |
| 4.8 | pOGA159 | 6314 | 6314POGAc4 |

The mutant strains shown in the table were selected from different mutants obtained in the transformation carried out as described herein. These strains were used in different vaccination and attenuation degree assays.

### Example 5.- Analysis of the mutant strains obtained by transformation with replicative plasmids

### Example 5.1.- Analysis of the mutant strains obtained by transformation with replicative plasmid pOG

The transformed strain in Example 4.6, 232POGc9, was used to confirm the presence of the plasmid in the cells recovered from the transformation of Friis agar plates and grown in FriisHS medium with 10 µg/ml gentamicin.

To that end, total DNA was purified from the cells. The DNA obtained was digested with restriction enzyme *Sca*I, which in this case results in the appearance of bands that can be easily identified in an agarose gel (Figure 4A).

The appearance of bands corresponding to the plasmid which clearly stand out over the genomic DNA background indicate that the clone analyzed contains the plasmid and that this plasmid stably replicates to give rise to 25-30 copies of plasmid per cell.

The analysis of the results shows that the replicative plasmids of the invention stably propagate to the progeny of the transformed bacteria.

### Example 5.2.- Analysis of the mutant strains obtained by transformation with replicative plasmid pOGCRE

The transformed strain in Example 4.7, 232POGCREc1, includes the gene coding for Cre protein.

To check whether the introduction of heterologous genes by means of replicative plasmids gave rise to the expression of corresponding proteins in a detectable manner in *Mhyo,* a total protein extraction from the clones recovered in the transformation assays with plasmid pOGCRE was analyzed by denaturing polyacrylamide gel electrophoresis and, subsequently, a Western-blot was performed to detect the presence of Cre recombinase by immunological methods (Figure 4B).

In this case, the Cre protein was only detected in the strain which has been transformed with plasmid pOGCRE, thus showing the usefulness of the replicative plasmids for the heterologous expression of proteins in *Mhyo.*

### Example 5.3.- Analysis of the mutant strains obtained by transformation with replicative plasmid pOGA159

The transformed strain in Example 4.8, 6314pOGAc4, was used to confirm the presence of the plasmid in the cells recovered from the transformation of Friis agar plates and grown in FriisHS medium with 0.5 µg/ml tetracycline.

To that end, total DNA was purified from the cells. The DNA obtained was analyzed by means of an agarose gel which in this case results in the appearance of bands that can be easily identified when comparing to the DNA preparation obtained from parental strain 6314 (Figure 4C).

### Example 6.- Analysis of the mutant strains obtained by transformation by means of transposition (not part of the invention)

### Example 6.1.- Analysis of the mutant strains obtained by transformation by means of transposition of plasmid pTC3

Different transformant clones obtained by electroporation with plasmid TC3 were analyzed both by Southern blot and by direct sequencing of the genomic DNA to show that the tetracycline-resistant mutants were the result of the presence of the transposon insertion in the bacterial chromosome.

To ensure that all the clones obtained were bearing a single transposon insertion and these insertions were not remobilized after several generations, a Southern blot of the genomic DNA of a clone obtained by means of plasmid pTC3 named 232TC3hlyC was performed. This clone was so called because the sequencing results indicated that the transposon had been inserted in the coding sequence of the *hlyC* gene (base 843448 of the genome of strain 232, GenBank AE017332.1). To that end, both the genomic DNA of strain 232 and that of strain 232TC3hlyC were digested with restriction enzymes EcoRI and EcoRV. The resulting fragments were separated in a 0.7% agarose gel and were transferred to a nylon membrane. A probe was generated with oligonucleotides, SondaTet-5 (ATG AGT GGA TCC ATG AAA ATT ATT AAT ATT G) (SEQ ID NO: 44) and SondaTet-3 (CTA AGT TAT TTT ATT GAA CAT ATA TCG TAC TTT ATC) (SEQ ID NO: 45), by means of a PCR reaction with digoxigenin-labeled dUTP using plasmid pTC3 as a template. The probe recognizes the tetracycline resistance gene sequence *TetM* so that the regions of the genome where insertion of the transposon occurred (Figure 5A) were detected in the Southern blot. The number and size of the bands coincided with those theoretically expected, indicating that the mutants obtained by this method and such vectors have a single copy of transposon insertion and that said insertion is kept stable after several generations.

### Example 6.2.- Analysis of the mutant strains obtained by transformation by means of transposition of plasmid pTC3C, pTC3L and pTC3T

Several mutants originated from each transposon were selected and called 6314Cc1, 232Cc6, 232Lc2, 232Tc2. As mentioned previously, two mutants with the plasmid designed for cytosolic expression (one, 6314Cc1, for strain 6314 and another, 232Cc6, for strain 232) were selected whereas the versions designed to be localized in the membrane have only been exemplified on strain 232 such as mutant 232Lc2 from plasmid pTC3L and mutant 232Tc2 from pTC3T.

The insertion points of the cytosolic version transposons (pTC3C) in the bacterial chromosome were determined by direct genomic DNA sequencing. Referring to the genome of strain 232 (GenBank AE017332.1) the transposon in clone 6314Cc1 was inserted in base 224547 whereas the transposon in clone 232Cc6 was inserted in base 569253.

To identify the presence of PCV2 capsid protein and to quantify it in the strains of *Mhyo* obtained (232Lc2, 232Cc6, 232Tc2 and 6314Cc1), a commercial kit (Ingenasa) which allows detecting and quantifying said antigen by means of on-plate immunodetection (ELISA) was used. The protein extracts were obtained after concentrating the cultures in exponential phase of each strain 100x and lysing the cells obtained in PBS and Triton^{®} X-100 at a final concentration of 0.1% (v/v). The results were positive indicating that all the tested strains and plasmids express the proteins coded by ORF2 or ORF2v2 to a greater or lesser amount (Figure 5B).

The strains with the version from plasmid pTC3C were also analyzed by means of denaturing polyacrylamide gel electrophoresis and Western-blot (Figure 5C). Bands of the same molecular weight as PCV2 capsid protein were detected. Interestingly, there was no proteolytic degradation band, a common problem in the recombinant protein expression. All this suggests that PCV2 capsid protein expressed in *Mhyo* in a recombinant manner can be a good antigen for formulating a polyvalent vaccine. Likewise, it is confirmed that *Mhyo* is a good host for expressing recombinant proteins.

### Example 7.- Vaccination assays (not part of the invention)

### Example 7.1.- Vaccination and challenge with PCV2

It was tested the efficacy of vaccines containing transformed strains of *Mhyo* prepared as described herein, wherein said strains expressed PCV2 circovirus capsid protein in their cytosol, and the animals were challenged with a pathogenic strain of PCV2.

The responses assessed were the immunological response against *Mhyo* and against PCV2 determined by means of ELISA and the PCV2 viral DNA load in the serum of the animals.

For the test 72 pigs of 28 days of age were selected, they were randomly divided into 6 groups of 12 animals each.

Said efficacy was tested according to a 2² factorial design with four groups of animals, in which the factors to be assessed were the transformed strains of *Mhyo* and the vaccination regimen. To that end vaccines containing strains 6314Cc1 or 232Cc6 were tested, and the animals were vaccinated following a single-dose regimen or revaccination regimen with an additional dose.

The assay groups performed are shown in Table II:

**Table II**

| Group | Revaccination | Strain |
|---|---|---|
| 1 | No | 6314Cc1 |
| 2 | Yes | 6314Cc1 |
| 3 | No | 232Cc6 |
| 4 | Yes | 232Cc6 |

Additionally, two groups of animals were incorporated in the study: a non-vaccinated but infected group (group 5), and a non-vaccinated and non-infected group (group 6).

The vaccines comprised the mutant strains of *Mhyo* inactivated by means of a non-ionic surfactant and a W/O/W-type emulsion as an adjuvant, and were administered intramuscularly on the neck of the animal.

The vaccination regimen consisted of administering a dose of 2 ml of vaccine at study day 0 to all the animals which were not part of the control groups, and half of the animals were revaccinated 14 days after first dose.

The animals in groups 5 and 6 received 2 ml of PBS as placebo both on the first day and on the 14^{th} day.

To prepare the antigens for the vaccines, transformed strains of *Mhyo* were grown in Friis medium with 0.5 µg/ml of tetracycline at a temperature of 37° C under shaking between 100 and 200 rpm until a change in color due to a change in pH of the culture medium was observed.

The cultures were centrifuged and washed twice with PBS to obtain a 30-times concentrated culture. The antigen corresponding to transformed *Mhyo* strain 6314Cc1 had a titer of 9.2 color changing units/ml (CCU/ml log₁₀) and the antigen corresponding to transformed *Mhyo* strain 232Cc6 has a titer of 9.45 CCU/ml log₁₀. PCV2 capsid protein production was confirmed by means of ELISA (Ingezim PCV DAS, Ingenasa, Spain). The total protein of each antigen was 373 mg/l and 350.2 mg/l, respectively, determined by the Coomassie blue method.

The antigens were inactivated with a non-ionic surfactant and a W/O/W-type emulsion, such as Montanide^{®} ISA 201 product (SEPPIC, France) in a ratio of 50:50 weight/weight, for example, was used as an adjuvant.

Blood samples were collected from the animals the day before the vaccination (day -1), day 13 (the day before revaccination), day 27 (before infection), and on day 7, 14, and 21 after the infection, corresponding respectively to day 35, 42 and 49 of the study.

Serum was obtained for determining the immunological response against PCV2 by means of the PCV2 Antibody ELISA test kit (Biocheck, The Netherlands) and against *Mhyo* by means of CIVTEST SUIS Myo (Hipra, Girona-Amer, Spain); and also for PCV2 viremia analysis.

The serum samples were obtained on days -1, 13 and 27 and, since they were expected to be negative, they were analyzed by standard PCR such as described, for example, in Quintana et al., Veterinary Record, 2001, 149, 357-361, whereas the samples collected on days 7 and 14 after infection were analyzed by quantitative PCR, as described, for example, in Olvera et al., J. Virol. Meth., 2004, 10 117, 75-80.

The animals from groups 1 to 5 were infected intranasally with 2 ml (5.66 CCID₅₀/ml log₁₀) of a virulent wild type strain of PCV2 (Sp-107-54-13 PCV2, Fort et al., Vet. Immunol. Immunopathol., 2010, 137, 226-234) 28 days after the administration of the first dose of the vaccine. The animals from group 6 received 2 ml of PBS intranasally as placebo. The animals were euthanized on day 49 of the study, i.e., 21 days after infection.

During the completely blind, randomized infection-vaccination trial, the animals were housed in rooms with biosafety level 3. The group 6 was placed in a separate room to prevent cross-infections and was therefore the only group which was unblinded for the staff responsible of taking care of the animals.

Taking into account that the animals had anti-PCV2 maternal antibodies, the groups were treated in blocks with relation to this parameter to prevent initial differences among the groups.

The primary parameter for evaluating the efficacy of PCV2 infection was the reduction of PCV2 viremia determined by quantitative PCR.

The primary parameter for determining the efficacy with relation to *Mhyo* was the sero-conversion analyzed by ELISA.

Serological response to *Mhyo* is shown in Figure 6. The four test vaccines promoted a significant sero-conversion compared with the non-vaccinated groups (groups 5 and 6) from day 13 of the study onwards.

After PCV2 infection, the vaccinated groups showed a greater immunological response with relation to the non-vaccinated but infected group on day 14 after infection and also on day 21 after infection with the exception of group 4.

The non-vaccinated and non-infected group showed the normal drop of anti-PCV2 maternal antibodies with a significant difference with the non-vaccinated and infected group on day 21 after infection.

No clinical signs associated with PCV2 infections were observed. PCV2 test models are normally sub-clinical models, and the key parameter for determining the vaccine efficacy is viremia reduction.

All the animals were negative with respect to PCV2 according to the analysis performed by standard PCR before infection. Figure 8 shows the genomic copies of PCV2 which were quantified by real time by PCR 7 and 14 days after infection. No genomic copies of PCV2 were detected in the serum samples of the non-vaccinated and non-infected group.

PCV2 viremia was significantly lower in the vaccinated groups compared with the non-vaccinated but infected group on day 14 after infection.

The results of this study indicate that the strains of *Mhyo* transformed by means as described herein and expressing the PCV2 capsid protein generate at the same time a significant immunological response against *Mhyo* and a porcine circovirus type 2 (PCV2).

The differential immunological response against PCV2 in vaccinated animals was clear on day 14 after infection, once PCV2 infection had been established, as inferred from the PCV2 viremia which is observed in the non-vaccinated and infected group (group 5).

Contrary to what happened with *Mhyo,* the presence of anti-PCV2 maternal antibodies prevented vaccine immunogenicity evaluation in the absence of an experimental infection.

Since the clinical signs corresponding to macroscopic lesions due to PCV2 infection cannot be reproduced in an experimental model, the result of PCV2 infection was assessed in terms of viremia by quantitative PCR and the vaccine efficacy was shown by means of viremia reduction. This reduction was observed for the four test vaccines 14 days after infection once the viremia reaches its highest level.

Therefore, the transformed strains of *Mhyo* expressing the PCV2 protein capsid can be used in vaccines against infections caused by *Mhyo* and by porcine circovirus type 2 (PCV2) at the same time.

### Example 7.2.- Vaccination and infection with PCV2 and Mhyo

It was tested the efficacy of a vaccine containing a transformed strain of *Mhyo* prepared as described herein, wherein said strain expressed PCV2 circovirus capsid protein in its cytosol. The animals were challenged with pathogenic strains of PCV2 and *Mhyo.*

The responses assessed were the PCV2 viral DNA load in the serum of the animals, the number of animals with PCV2 viremia, and Mhyo-like lesions in the lungs of animals.

For the test 36 pigs of 28 days of age were selected, they were randomly divided into 3 groups of 12 animals each.

Said efficacy was tested with a vaccine containing strain 6314Cc1. The animals of group 1 were vaccinated following a single-dose regimen. Additionally, two groups of animals were incorporated in the study: a non-vaccinated but infected group (group 2), and a non-vaccinated and non-infected group (group 3).

The vaccine comprised the mutant strain of *Mhyo* inactivated by means of a non-ionic surfactant and a W/O/W-type emulsion as an adjuvant, and was administered intramuscularly on the neck of the animal.

The vaccination regimen consisted of administering a dose of 2 ml of vaccine at study day 0 to all the animals which were not part of the control groups.

The animals in groups 2 and 3 received 2 ml of PBS as placebo.

To prepare the antigens for the vaccine, the transformed strain of *Mhyo* was grown in Friis medium with 0.5 µg/ml of tetracycline at a temperature of 37° C under shaking between 100 and 200 rpm until a change in color due to a change in pH of the culture medium was observed.

The cultures were centrifuged and resuspended in PBS to obtain a 25-times concentrated culture. The antigen corresponding to transformed *Mhyo* strain 6314Cc1 had a titer of 9,325 color changing units/ml (CCU/ml log₁₀). PCV2 capsid protein production was confirmed by means of ELISA (Ingezim PCV DAS, Ingenasa, Spain).

The antigens were inactivated with a non-ionic surfactant, and as adjuvant it was used a W/O/W-type emulsion, for example, the product Montanide^{®} ISA 201 (SEPPIC, France) in a ratio of 50:50 weight/weight.

Blood samples were collected from the animals the day before the vaccination (day -1), on day 13, 21 and 27 after vaccination, and on day 7, 14, 21 and 28 after the infection, corresponding respectively to day 35, 42, 49 and 56 of the study.

Serum was obtained for determining PCV2 viremia by using quantitative PCR, as described, for example, in Olvera *et al.,* already cited.

The animals from groups 1 and 2 were infected intranasally with 2 ml/animal (5.33 CCID₅₀/ml log₁₀) of a virulent wild type strain of PCV2 (Sp-107-54-13 PCV2, Fort et al., Vet. Immunol. Immunopathol., 2010, 137, 226-234), and intratracheally with 10 ml/animal of the pathogenic strain *Mhyo* 3371 (7.325 CCU/ml log₁₀) 28 days after the administration of the vaccine. The animals from group 3 received 2 ml of PBS intranasally and 10 ml PBS/animal intratracheally as placebo. The animals were euthanized on day 56 of the study, i.e., 28 days after infection, at that moment the presence of Mhyo-like lesions in the lungs of animals was assessed.

During the completely blind, randomized infection-vaccination trial, the animals were housed in rooms with biosafety level 3. The group 3 was placed in a separate room to prevent cross-infections and was therefore the only group which was unblinded for the staff responsible of taking care of the animals.

Taking into account that the animals had anti-PCV2 maternal antibodies, the groups were treated in blocks with relation to this parameter to prevent initial differences among the groups.

The primary parameter for evaluating the efficacy of PCV2 infection was the reduction of PCV2 viremia determined by quantitative PCR. The primary parameter for determining the efficacy with relation to *Mhyo* was the lung surface affected by Mhyo-like lesions.

All the animals were negative with respect to PCV2 according to the analysis performed by standard PCR before the experimental infection. Figure 12 shows the genomic copies of PCV2 which were quantified by real time by PCR 7, 14, 21 and 28 days after infection. No genomic copies of PCV2 were detected in the serum samples of the non-vaccinated and non-infected group. PCV2 viremia was significantly lower in the vaccinated group compared with the non-vaccinated but infected group on days 14 and 21 after infection.

In Figure 13 are shown the same results expressed as percentage of animals with viremia (positive in quantitative PCR).

In Figure 14 it is shown the median of the percentage of lung surface affected by Mhyo-like lesions. It was observed that the affected lung surface in vaccinated animals was significantly lower than that of non-vaccinated but infected animals (group 2).

The results of this study led to the conclusion that the strain of *Mhyo* genetically transformed as described herein and expressing the PCV2 capsid protein, generated at the same time a significant reduction in the PCV2 viremia and in the Mhyo-like lesions in comparison with the non-vaccinated animals.

Consequently, the transformed strains of *Mhyo* expressing the PCV2 protein capsid can be used in vaccines against infections caused by *Mhyo* and by porcine circovirus type 2 (PCV2) at the same time.

### Example 8.- Tests of the degree of attenuation of transformed mutant strains

### Example 8.1.- Testing the degree of attenuation of a transformed mutant strain bearing transposon insertion in Mhyo hemolysin C gene (not part of the invention)

The degree of attenuation of a mutant strain of *Mhyo* obtained by transposition was determined in this test by means of assessing the ability of said strain to colonize the upper and lower respiratory tract of pigs.

The tested mutant strain, 232TC3hlyC, obtained in Example 4.5, shows transposon insertion in *Mhyo* hemolysin C gene (*hlyC*).

Pigs of 8 weeks of age were selected, they were randomly divided into 2 groups of 8 animals each, and each group was kept in separate rooms to prevent cross-infections.

The animals were sedated before being infected. A group of animals was infected intratracheally on day 0 with 10 ml of mutant strain 232TC3hlyC, and the other group with 10 ml of the parental strain 232 of *Mhyo.* The inoculums had a titer of 7 CCU/ml log₁₀. The animals were euthanized on day 28 of the study.

Nasal samples were collected on days -1 (the day before infection), 8, 15, 21 and 28 to perform a *Mhyo* analysis by nested PCR.

Bronchial samples were collected on day 28 days from euthanized animals to perform a *Mhyo* analysis also by nested PCR.

No animals bearing the genomic DNA of *Mhyo* were detected in the nasal samples collected throughout the course of the study. However, *Mhyo* wild type strain 232 was detected in 25% of the bronchial samples of the animals, whereas the detection rate was 0% for the transformed mutant strain.

This result suggests that the mutant strain 232TC3hlyC shows an attenuated behavior in comparison with the parental strain which it comes from, in relation to lower respiratory tract colonization; therefore it can be used as an attenuated candidate in a vaccine against *Mhyo.*

### Example 8.2.- Testing the degree of attenuation of a transformed mutant strain showing inhibition of the expression of Mhyo hemolysin 159 gene

The degree of attenuation of the strain 6314POGAc4 obtained in Example 4.8 showing inhibition of the expression of *Mhyo* hemolysin 159 gene was determined in another test.

Pigs of 8 weeks of age were selected, they were randomly divided into 3 groups of 8 animals each and were kept in separate rooms to prevent cross-infections.

On day 0 of the test, the animals from group 1 were intratracheally inoculated with 10 ml of strain 6314POGAc4 with a concentration of 8 CCU/ml log₁₀, and the same amount of wild type parental strain 6314 was administered to the animals from group 2. The animals were sedated before being infected. Group 3 was the non-infected control group. The animals were euthanized on day 28 of the study.

Blood samples were collected the day before the infection (day -1), and on days 15 and 28. *Mhyo* serology was analyzed by means of CIVTEST SUIS Mhyo (Hipra-Amer-Girona-Spain). Nasal samples were collected on days -1, 8, 15, 21 and 28 to perform a *Mhyo* analysis by nested PCR.

Bronchial samples were collected on day 28 from euthanized animals to perform a *Mhyo* analysis also by nested PCR.

The macroscopic catarrhal bronchopneumonia lung lesions compatible with a *Mhyo* infection were rated. Each lung lobe was rated from 0 and 5 according to the proportion of tissue with lesions caused by *Mhyo*.

The contribution of each lung lobe to the total affected lung surface was calculated by applying the method described in Christensen et al., Diseases of the respiratory system. In: Diseases of Swine. 8th Edition. Straw B.E., D'Allaire S., Mengeling W.L. and Taylor D.J. Iowa State University Press, Ames (IA) 1999, page 914.

Figure 9 shows the serological response against *Mhyo* infection. It can be observed that only the wild type parental strain (group 2) showed sero-conversion 28 days after infection, with a significant difference with respect to group 1, infected with transformed mutant strain 6314POGAc4, and to the control group.

Figure 10 shows that *Mhyo* was only detected in the nasal samples of group 2 21 and 28 days after infection. In the 8 animals of group 2 *Mhyo* was detected in the bronchial samples collected on day 28, whereas the number was significantly less in the group to which the transformed mutant strain of the invention was administered.

It can be observed in Figure 11 that the macroscopic lung lesions caused by *Mhyo* were significantly higher in group 2, infected with the wild type parental strain, whereas the animals infected with the mutant strain of the invention showed smaller lesions comparable to those observed in the non-infected group.

Therefore, it can be concluded that the transformed mutant strain of the invention tested in this example shows a reduced ability to colonize the upper and lower respiratory tract and also a reduced capacity to cause PEP-associated lung lesions.

All this suggests that said transformed mutant strain can be used for preparing attenuated vaccines against PEP and *Mhyo*-associated pathologies.

## Claims

1. A method for preparing a mutant strain of *Mycoplasma hyopneumoniae (Mhyo),* **characterized in that** it comprises the step of transforming a strain of *M. hyopneumoniae* by means of using a carrier vector comprising at least one exogenous DNA sequence, that sequence being under the control of a DNA sequence of a promoter region of *M. hyopneumoniae,*
wherein the carrier vector is a replicative plasmid vector comprising:
1) a DNA sequence comprising the *oriC* region of a strain of *Mycoplasma sp., which is Mycoplasma hyopneumoniae,* and
2) an exogenous DNA sequence comprising a marker gene, and optionally, an additional exogenous DNA sequence, which are under the control of a DNA sequence of a promoter region of *Mhyo,*
wherein the promoter region of *Mhyo* corresponds to a DNA segment comprising between 50 base pairs and 300 base pairs located on the 5' side of the gene of a *Mhyo* protein selected from the group formed by the P36, P46, P65, P76, P97, P102, P146, and P216 proteins,
wherein the promoter region of *Mhyo* initiates or promotes the transcription of a *Mhyo* DNA sequence, and
wherein the exogenous DNA sequence is stably introduced in the cytosol of the strain of *Mhyo.*

2. The method according to claim 1, **characterized in that** the exogenous DNA sequence is selected from the group comprising a resistance gene to an antibiotic, a gene coding for a recombinase, a DNA fragment from *Mhyo,* a gene coding for an antigenic component of a microorganism causing porcine diseases, and combinations thereof.

3. The method according to any one of claims 1 or 2, **characterized in that** the additional exogenous DNA sequence is selected from the group formed by the DNA sequence coding for the protein of the capsid of the porcine circovirus type 2 (PCV2) and the DNA sequence coding for a protein comprising the protein of the capsid of the porcine circovirus type 2 (PCV2) additionally bearing MetSerGlySer amino acids at the N-terminal end of said protein, said additional exogenous DNA sequence coding for the protein of the PCV2 capsid being selected from the group formed by SEQ ID NO: 3, SEQ ID NO: 4, and SEQ ID NO: 5.

4. The method according to any one of claims 1 to3, **characterized in that** the promoter region of *Mhyo* comprises the promoter region of the gene of a *Mhyo* protein selected from the group formed by the P46 and P97 proteins.

5. The method according to claim 4, **characterized in that** the promoter region of *Mhyo* comprises the promoter region of the gene of the P46 *Mhyo* protein.

6. The method according to claim 4, **characterized in that** the promoter region of *Mhyo* comprises the promoter region of the gene of the P97 *Mhyo* protein.

7. A replicative plasmid vector, **characterized in that** it comprises:
1) a DNA sequence comprising the *oriC* region of a strain of *Mycoplasma sp.,* which is *Mycoplasma hyopneumoniae,* and
2) an exogenous DNA sequence comprising a marker gene, and optionally an additional exogenous DNA sequence which are under the control of a DNA sequence of a promoter region of *Mhyo,*
wherein the promoter region of *Mhyo* corresponds to a DNA segment comprising between 50 base pairs and 300 base pairs located on the 5' side of the gene of a *Mhyo* protein selected from the group formed by the P36, P46, P65, P76, P97, P102, P146, and P216 proteins, and
wherein the promoter region of *Mhyo* initiates or promotes the transcription of a *Mhyo* DNA sequence.

8. The replicative plasmid vector according to claim 7, **characterized in that** the additional exogenous DNA sequence is selected from the group formed by the sequence coding for the capsid protein of porcine circovirus type 2 (PCV2) and the sequence coding for a protein comprising the capsid protein of the porcine circovirus type 2 (PCV2) additionally bearing MetSerGlySer amino acids in the N-terminal end of said protein, said DNA sequence coding for the PCV2 capsid protein is selected from the group formed by SEQ ID NO: 3, SEQ ID NO: 4, and SEQ ID NO: 5.

9. The replicative plasmid vector according to any one of claims 7 or 8, **characterized in that** the promoter region of *Mhyo* comprises the promoter region of the gene of a *Mhyo* protein selected from the group formed by the P46 and P97 proteins.

10. A mutant strain of *Mhyo,* **characterized in that** it comprises the replicative plasmid vector according to any one of claims 7 to 9.

11. A vaccine for protecting pigs against porcine enzootic pneumonia caused by *Mhyo,* and optionally against another disease or additional pathological conditions affecting pigs, comprising an immunologically effective amount of the mutant strain of *Mhyo* as defined in claim 10.

12. The vaccine according to claim 11, **characterized in that** the another disease or additional pathological conditions affecting pigs is caused by a microorganism, which is selected from the group formed by *Actinobacillus sp., Brachyspira sp., Pasteurella multocida, Salmonella sp., Streptococcus sp., Isospora sp., Erysipelothrix rhusiopathiae, Leptospira sp., Staphylococcus sp., Haemophilus parasuis, Bordetella bronchiseptica, Clostridium sp., Mycoplasma sp., Lawsonia intracellularis, Escherichia coli,* porcine reproductive and respiratory syndrome virus, swine influenza virus, contagious gastroenteritis virus, porcine parvovirus, encephalomyocarditis virus, coronavirus, rotavirus, porcine circovirus, porcine periweaning failure to thrive syndrome agent, classical swine fever virus, African swine fever virus, calicivirus and torque teno virus (TTV).

13. The vaccine according to claim 12, **characterized in that** the another disease or additional pathological conditions affecting pigs is caused by porcine circovirus.

14. The vaccine according to any one of claims 11 to 13, **characterized in that** it further comprises a pharmaceutically acceptable vehicle, and optionally an adjuvant.

15. A vaccination kit for vaccinating pigs against an infection or disease caused by *Mhyo* and optionally against another disease or pathological conditions caused by microorganisms affecting pigs, comprising a container comprising an immunologically effective amount of the mutant strain as defined in claim 10.

## Patentansprüche

1. Verfahren zur Herstellung eines Mutantenstammes von *Mycoplasma hyopneumoniae (Mhyo),* dadurch charakterisiert, dass es den Schritt des Transformierens eines Stammes von *M. hyopneumoniae* unter Verwendung eines Trägervektors umfasst, welcher mindestens eine exogene DNA-Sequenz umfasst, wobei diese Sequenz unter der Kontrolle einer DNA-Sequenz einer Promotorregion von *M. hyopneumoniae* steht,
wobei es sich bei dem Trägervektor um einen replikativen Plasmidvektor handelt, umfassend:
1) eine DNA-Sequenz umfassend die *oriC*-Region eines Stammes von *Mycoplasma sp*., wobei es sich um *Mycoplasma hyopneumoniae* handelt, und
2) eine exogene DNA-Sequenz umfassend ein Marker-Gen, und optional eine zusätzliche exogene DNA-Sequenz, welche unter der Kontrolle einer DNA-Sequenz einer Promotorregion von *Mhyo* stehen,
wobei die Promotorregion von *Mhyo* einem DNA-Segment entspricht, welches zwischen 50 Basenpaare und 300 Basenpaare umfasst, welche auf der 5'-Seite des Gens eines Mhyo-Proteins ausgewählt aus der Gruppe bestehend aus den Proteinen P36, P46, P65, P76, P97, P102, P146 und P216 lokalisiert sind,
wobei die Promotorregion von *Mhyo* die Transkription einer *Mhyo* DNA-Sequenz initiiert oder fördert, und
wobei die exogene DNA-Sequenz stabil in das Cytosol des Stammes von *Mhyo* eingebracht wird.

2. Verfahren nach Anspruch 1, dadurch charakterisieret, dass die exogene DNA-Sequenz ausgewählt ist aus der Gruppe umfassend ein Resistenz-Gen gegen ein Antibiotikum, ein Gen welches eine Rekombinase kodiert, ein DNA-Fragment von *Mhyo,* ein Gen welches eine antigene Komponente eines Schweinekrankheiten verursachenden Mikroorganismus kodiert, und Kombinationen davon.

3. Verfahren nach einem der Ansprüche 1 oder 2, dadurch charakterisiert, dass die zusätzliche exogene DNA-Sequenz ausgewählt ist aus der Gruppe bestehend aus der DNA-Sequenz, welche das Capsid-Protein des Schweine-Zirkovirus Typ 2 (PCV2) kodiert, und der DNA-Sequenz, welche ein Protein kodiert umfassend das Capsid-Protein des Schweine-Zirkovirus Typ 2 (PCV2) mit den zusätzlichen Aminosäuren MetSerGlySer am N-terminalen Ende des Proteins, wobei die zusätzliche exogene DNA-Sequenz, welche das PCV2 Capsid-Protein kodiert, ausgewählt ist aus der Gruppe bestehend aus SEQ ID NO: 3, SEQ ID NO: 4, und SEQ ID NO: 5.

4. Verfahren nach einem der Ansprüche 1 bis 3, dadurch charakterisiert, dass die Promotorregion von *Mhyo* die Promotorregion des Gens eines *Mhyo*-Proteins ausgewählt aus der Gruppe bestehend aus den Proteinen P46 und P97 umfasst.

5. Verfahren nach Anspruch 4, dadurch charakterisiert, dass die Promotorregion von *Mhyo* die Promotorregion des Gens des *Mhyo*-Proteins P46 umfasst.

6. Verfahren nach Anspruch 4, dadurch charakterisiert, dass die Promotorregion von *Mhyo* die Promotorregion des Gens des *Mhyo*-Proteins P97 umfasst.

7. Replikativer Plasmidvektor, dadurch charakterisiert, dass er umfasst:
1) eine DNA-Sequenz umfassend die *oriC*-Region eines Stammes von *Mycoplasma sp*., wobei es sich um *Mycoplasma hyopneumoniae* handelt, und
2) eine exogene DNA-Sequenz umfassend ein Marker-Gen, und optional eine zusätzliche exogene DNA-Sequenz, welche unter der Kontrolle einer DNA-Sequenz einer Promotorregion von *Mhyo* stehen,
wobei die Promotorregion von *Mhyo* einem DNA-Segment entspricht, welches zwischen 50 Basenpaare und 300 Basenpaare umfasst, welche auf der 5'-Seite des Gens eines *Mhyo*-Proteins ausgewählt aus der Gruppe bestehend aus den Proteinen P36, P46, P65, P76, P97, P102, P146 und P216 lokalisiert sind,
wobei die Promotorregion von *Mhyo* die Transkription einer *Mhyo* DNA-Sequenz initiiert oder fördert.

8. Replikativer Plasmidvektor nach Anspruch 7, dadurch charakterisiert, dass die zusätzliche exogene DNA-Sequenz ausgewählt ist aus der Gruppe bestehend aus der DNA-Sequenz, welche das Capsid-Protein des Schweine-Zirkovirus Typ 2 (PCV2) kodiert, und der DNA-Sequenz, welche ein Protein kodiert umfassend das Capsid-Protein des Schweine-Zirkovirus Typ 2 (PCV2) mit den zusätzlichen Aminosäuren MetSerGlySer am N-terminalen Ende des Proteins, wobei die DNA-Sequenz, welche das PCV2 Capsid-Protein kodiert, ausgewählt ist aus der Gruppe bestehend aus SEQ ID NO: 3, SEQ ID NO: 4, und SEQ ID NO: 5.

9. Replikativer Plasmidvektor nach einem der Ansprüche 7 oder 8, dadurch charakterisiert, dass die Promotorregion von *Mhyo* die Promotorregion des Gens eines *Mhyo-*Proteins ausgewählt aus der Gruppe bestehend aus den Proteinen P46 und P97 umfasst.

10. Mutantenstamm von *Mhyo,* dadurch charakterisiert, dass er den replikativen Plasmidvektor nach einem der Ansprüche 7 bis 9 umfasst.

11. Impfstoff zum Schutz von Schweinen gegen durch *Mhyo* verursachte enzootische Schweine-Pneumonie, und optional gegen eine andere Krankheit oder zusätzliche pathologische Zustände bei Schweinen, umfassend eine immunologisch wirksame Menge des wie in Anspruch 10 definierten Mutantenstammes von *Mhyo.*

12. Impfstoff nach Anspruch 11, dadurch charakterisiert, dass die andere Krankheit oder die zusätzlichen pathologischen Zustände bei Schweinen durch einen Mikroorganismus verursacht werden, welcher ausgewählt ist aus der Gruppe bestehend aus *Actinobacillus sp., Brachyspira sp., Pasteurella multocida, Salmonella sp., Streptococcus sp., Isospora sp., Erysipelothrix rhusiopathiae, Leptospira sp., Staphylococcus sp., Haemophilus parasuis, Bordetella bronchiseptica, Clostridium sp., Mycoplasma sp., Lawsonia intracellularis, Escherichia coli,* Schweine Reproduktions- und Atemwegs-Syndrom Virus, Schweine-Influenza Virus, ansteckendes Gastroenteritis-Virus, Schweine-Parvovirus, Enzephalomyokarditis-Virus, Coronavirus, Rotavirus, Schweine-Zirkovirus, Schweine Periweaning Failure to Thrive Syndrom-Agens, klassisches Schweinefieber-Virus, Afrikanisches Schweinefieber-Virus, Calicivirus und Torque Teno Virus (TTV).

13. Impfstoff nach Anspruch 12, dadurch charakterisiert, dass die andere Krankheit oder die zusätzlichen pathologischen Zustände bei Schweinen durch Schweine-Zirkovirus verursacht werden.

14. Impfstoff nach einem der Ansprüche 11 bis 13, dadurch charakterisiert, dass er weiterhin einen pharmazeutisch verträglichen Träger, und optional ein Adjuvans, umfasst.

15. Impf-Kit zur Impfung von Schweinen gegen eine durch *Mhyo* verursachte Infektion oder Krankheit, und optional gegen eine andere Krankheit oder pathologische Zustände, welche von Schweine befallenden Mikroorganismen verursacht werden, umfassend einen Behälter umfassend eine immunologisch wirksame Menge des wie in Anspruch 10 definierten Mutantenstammes.

## Revendications

1. Procédé de préparation d'une souche mutante de *Mycoplasma hyopneumoniae (Mhyo),*
**caractérisé en ce qu'**il comprend l'étape de transformation d'une souche de *M. hyopneumoniae* au moyen de l'utilisation d'un vecteur transporteur comprenant au moins une séquence d'ADN exogène, laquelle séquence étant sous le contrôle d'une séquence d'ADN d'une région promotrice de *M. hyopneumoniae,*
dans lequel le vecteur transporteur est un vecteur plasmidique réplicatif comprenant :
1) une séquence d'ADN comprenant la région *oriC* d'une souche de *Mycoplasma sp., qui est Mycoplasma hyopneumoniae,* et
2) une séquence d'ADN exogène comprenant un gène marqueur, et facultativement, une séquence d'ADN exogène supplémentaire, qui sont sous le contrôle d'une séquence d'ADN d'une région promotrice de *Mhyo,*
dans lequel la région promotrice de *Mhyo* correspond à un segment d'ADN comprenant entre 50 paires de bases et 300 paires de bases situées du côté 5' du gène d'une protéine *Mhyo* sélectionnée dans le groupe formé par les protéines P36, P46, P65, P76, P97, P102, P146, et P216,
dans lequel la région promotrice de *Mhyo* initie ou active la transcription d'une séquence d'ADN de *Mhyo,* et
dans lequel la séquence d'ADN exogène est introduite de manière stable dans le cytoplasme de la souche de *Mhyo.*

2. Procédé selon la revendication 1, **caractérisé en ce que** la séquence d'ADN exogène est sélectionnée dans le groupe comprenant un gène de résistance à un antibiotique, un gène codant une recombinase, un fragment d'ADN provenant de *Mhyo,* un gène codant un constituant antigénique d'un microorganisme provoquant des maladies porcines, et des combinaisons de ceux-ci.

3. Procédé selon l'une quelconque des revendications 1 ou 2, **caractérisé en ce que** la séquence d'ADN exogène supplémentaire est sélectionnée dans le groupe formé par la séquence d'ADN codant la protéine de la capside du circovirus porcin de type 2 (PCV2) et la séquence d'ADN codant une protéine comprenant la protéine de la capside du circovirus porcin de type 2 (PCV2) portant en plus les acides aminés MetSerGlySer à l'extrémité N-terminale de ladite protéine, ladite séquence d'ADN exogène supplémentaire codant la protéine de la capside de PCV2 étant sélectionnée dans le groupe formé par SEQ ID NO: 3, SEQ ID NO: 4, et SEQ ID NO: 5.

4. Procédé selon l'une quelconque des revendications 1 à 3, **caractérisé en ce que** la région promotrice de *Mhyo* comprend la région promotrice du gène d'une protéine *Mhyo* sélectionnée dans le groupe formé par les protéines P46 et P97.

5. Procédé selon la revendication 4, **caractérisé en ce que** la région promotrice de *Mhyo* comprend la région promotrice du gène de la protéine *Mhyo* P46.

6. Procédé selon la revendication 4, **caractérisé en ce que** la région promotrice de *Mhyo* comprend la région promotrice du gène de la protéine *Mhyo* P97.

7. Vecteur plasmidique réplicatif, **caractérisé en ce qu'**il comprend :
1) une séquence d'ADN comprenant la région *oriC* d'une souche de *Mycoplasma sp.,* qui est *Mycoplasma hyopneumoniae,* et
2) une séquence d'ADN exogène comprenant un gène marqueur, et facultativement une séquence d'ADN exogène supplémentaire qui sont sous le contrôle d'une séquence d'ADN d'une région promotrice de *Mhyo,*
dans lequel la région promotrice de *Mhyo* correspond à un segment d'ADN comprenant entre 50 paires de bases et 300 paires de bases situées du côté 5' du gène d'une protéine *Mhyo* sélectionnée dans le groupe formé par les protéines P36, P46, P65, P76, P97, P102, P146, et P216, et
dans lequel la région promotrice de *Mhyo* initie ou active la transcription d'une séquence d'ADN de *Mhyo.*

8. Vecteur plasmidique réplicatif selon la revendication 7, **caractérisé en ce que** la séquence d'ADN exogène supplémentaire est sélectionnée dans le groupe formé par la séquence codant la protéine de la capside du circovirus porcin de type 2 (PCV2) et la séquence codant une protéine comprenant la protéine de la capside du circovirus porcin de type 2 (PCV2) portant en plus les acides aminés MetSerGlySer à l'extrémité N-terminale de ladite protéine, ladite séquence d'ADN codant la protéine de la capside de PCV2 étant sélectionnée dans le groupe formé par SEQ ID NO: 3, SEQ ID NO: 4, et SEQ ID NO: 5.

9. Vecteur plasmidique réplicatif selon l'une quelconque des revendications 7 ou 8, **caractérisé en ce que** la région promotrice de *Mhyo* comprend la région promotrice du gène d'une protéine *Mhyo* sélectionnée dans le groupe formé par les protéines P46 et P97.

10. Souche mutante de *Mhyo,* **caractérisée en ce qu'**elle comprend le vecteur plasmidique réplicatif selon l'une quelconque des revendications 7 à 9.

11. Vaccin destiné à protéger les porcs contre la pneumonie enzootique du porc provoquée par *Mhyo,* et facultativement contre d'autres maladies ou états pathologiques supplémentaires touchant les porcs, comprenant une quantité immunologiquement efficace de la souche mutante de *Mhyo* telle que définie dans la revendication 10.

12. Vaccin selon la revendication 11, **caractérisé en ce que** les autres maladies ou états pathologiques supplémentaires touchant les porcs sont provoqués par un microorganisme, qui est sélectionné dans le groupe formé par *Actinobacillus sp., Brachyspira sp., Pasteurella multocida, Salmonella sp., Streptococcus sp., Isospora sp., Etysipelothrix rhusiopathiae, Leptospira sp., Staphylococcus sp., Haemophilus parasuis, Bordetella bronchiseptica, Clostridium sp., Mycoplasma sp., Lawsonia intracellularis, Escherichia coli,* le virus du syndrome dysgénésique et respiratoire porcin, le virus de la grippe porcine, le virus de la gastroentérite transmissible, le parvovirus porcin, le virus de l'encéphalomyocardite, le coronavirus, le rotavirus, le circovirus porcin, l'agent du PFTS (pour *porcine periweaning failure to thrive syndrome,* ou syndrome du dépérissement du porcelet en post-sevrage), le virus de la peste porcine classique, le virus de la peste porcine africaine, le calicivirus et le Torque Teno Virus (TTV).

13. Vaccin selon la revendication 12, **caractérisé en ce que** les autres maladies ou états pathologiques supplémentaires touchant les porcs sont provoqués par le circovirus porcin.

14. Vaccin selon l'une quelconque des revendications 11 à 13, **caractérisé en ce qu'**il comprend en outre un véhicule pharmaceutiquement acceptable, et facultativement un adjuvant.

15. Trousse de vaccination pour la vaccination des porcs contre une infection ou maladie provoquée par *Mhyo* et facultativement contre d'autres maladies ou états pathologiques provoqués par des microorganismes touchant les porcs, comprenant un récipient comprenant une quantité immunologiquement efficace de la souche mutante telle que définie dans la revendication 10.
